# EUROPEAN PATENT APPLICATION

(11) **EP 4 835 056 A1**
(43) Date of publication of application: **04.11.2026**
(21) Application number: 24912694.7
(22) Date of filing: 23.12.2024
(51) Int. Cl.: C12N 5/0783, C07K 14/47, C12N 1/00, C12N 5/10, C12N 5/0781

(54) **CELL CULTURE METHOD**

(30) Priority: 25.12.2023 JP 2023217677
(71) Applicant: Takeda Pharmaceutical Company Limited, Osaka-shi, Osaka 541-0045 (JP)
(72) Inventor: KUWAE, Shinobu, Fujisawa-shi, Kanagawa 251-0012 (JP)
(74) Representative: Watermeyer, Nicholas
(86) International application number: PCT/JP2024/045351
(87) International publication number: WO 2025/142807

(57) **Abstract**

The present invention aims to provide a method that makes it possible to sufficiently proliferate cells, particularly immunocompetent cells, even in a serum-free medium or low-serum medium, and a medium composition to be used for the method. An effect of maintaining and promoting cell proliferation comparable to that achieved by culture in a serum-containing medium can be obtained even in a serum-free medium or a low-serum medium, by containing imidazole dipeptide or a salt thereof, taurine or a precursor thereof or a salt thereof, and lysophosphatidic acid or a derivative thereof.

## Description

### [Technical Field]

The present invention relates to a method for culturing/proliferating cells in a serum-free medium or low-serum medium, and a medium composition for said method. The present invention relates to a method for producing a cell population in which the number of desired cells is expanded in a serum-free or low-serum medium, and a medicament containing the cell population obtained by said method.

### (Background of the Invention)

Conventionally, cell culture has been performed using a medium containing serum (hereinafter also to be referred to as "serum-containing medium"). For example, fetal bovine serum (FBS) is widely used in cell culture as an important additive for cell proliferation. However, when cultured cells or derivatives thereof are used for medical purposes, xenogeneic components may become a source of infection for blood-borne pathogens or heterologous antigens. In addition, differences between serum lots may cause variations in culture results. For this reason, in recent years, it has become mainstream to reduce as much as possible the amount of serum to be used and to culture cells using a medium with a clear chemical composition (chemically defined medium), and the development of serum-free medium and low-serum medium is underway.

Recently, chimeric antigen receptor (CAR) T cell (hereinafter to be simply referred to as CAR-T cell) therapy has been developed as one of the immunotherapies for cancer patients, in which the T cell receptor (TCR) of cytotoxic T cells (CTL) is genetically modified to make CTL directly and selectively recognize tumor cells, and an antitumor effect is exerted. Cancer therapy using CAR-T cells kills cancer cells based on a mechanism different from that of conventional anticancer drugs and radiation therapy, and is therefore expected to be effective against intractable or treatment-resistant cancers. Current CAR-T cell therapy is generally performed using autologous CAR-T cell therapy, in which CAR genes are introduced ex vivo into T cells collected from a patient, and the CAR-T cells produced thereby are then administered to the patient, as in Kymriah (trade name) and Yescarta (trade name) approved in the United States. However, this method requires multiple steps over a long period of time, such as activation/proliferation of T cells, preparation of viral vectors, and gene transfer into T cells, and therefore has the problem of high manufacturing costs due to the costs required for cell culture and preparation of viral vectors. Therefore, iPS cell-derived CAR-T cells that enable allogeneic CAR-T therapy have been developed. In this method, too, a serum-containing medium is used in the process of producing iPS cell-derived CAR-T cells, particularly in the expansion culture process, in order to achieve a sufficient cell proliferation effect. However, from the viewpoint of GMP (Good Manufacturing Practice), the medium is preferably xeno-free, i.e., not containing xenogeneic components, and desirably serum-free or low-serum. In addition, considering that T cells derived from iPS cells are particularly vulnerable to washing stress compared to primary T cells, there has been a strong demand for a serum-free or low-serum medium that does not require extensive washing.

There have been many reports on methods for maintaining desired cell proliferation effects or promoting cell proliferation effects in cell culture.

Non Patent Literature 1 describes that lysophosphatidylcholine (LPC) and α-tocopherol are useful for mouse spermatogenesis in a synthetic medium that does not contain albumin. Non Patent Literature 2 describes that carnosine is effective in extending the survival period of cells in in vitro culture of human peripheral blood-derived CD4⁺T cells. Non Patent Literature 3 describes that LPC has a cell proliferation effect in culture of CHO cells in a protein-free medium. Non Patent Literature 4 describes that α-tocopherol, together with selenium, is involved in the defense of cells against oxidative stress. Patent Literature 1 describes that the Hayflick limit of cells during culture can be increased by applying carnosine to cells. Patent Literature 2 describes that a serum-free medium containing taurine, carnosine, and vitamin E (α-tocopherol) can be used to improve the metabolic maturity state of cells. Non Patent Literature 5 describes that the addition of lysophosphatidic acid (LPA) is effective in promoting cell proliferation of CHO cells in a serum-free medium.

There is also a report on the cell proliferation effect of boric acid via its transporter NaBC1 (Non Patent Literature 6). Patent Literature 3 describes a serum-free medium containing borate and suitable for culturing diploid cells. Non Patent Literature 7 describes that Taut, which is a taurine transporter, is important for the recall response of T cells.

### [Citation List]

### [Patent Literature]

[Patent Literature 1]
   European Patent No. 0571390
[Patent Literature 2]
   JP 2014-521337 A
[Patent Literature 3]
   Chinese Patent No. 105462912

### [Non Patent Literature]

[Non Patent Literature 1]
   Sanjo, Hiroyuki et al., FASEB J. 2020 Jul; 34(7):9480-9497.
[Non Patent Literature 2]
   Hyland P et al., Mech Ageing Dev. 2000 Dec 20; 121(1-3):203-15.
[Non Patent Literature 3]
   In-Kyoung Kim et al., Biotechnol Bioprocess Eng. 2008; 13:396-400.
[Non Patent Literature 4]
   Saito, Yoshiro et al., J Biol Chem. 2003 Oct 10; 278(41):39428-34.
[Non Patent Literature 5]
   Miki, Hideo et al., Cytotechnology. 2015 Aug; 67(4):689-97.
[Non Patent Literature 6]
   Park, Meeyoung et al., Mol Cell. 2004 Nov 5; 16(3):331-41.
[Non Patent Literature 7]
   Kaesler, Susanne et al., Eur. J. Immunol. 2012 42:831-841.

### [Summary of Invention]

### [Technical Problem]

The present invention aims to provide a method for culturing and sufficiently proliferating cells, particularly immunocompetent cells, in a serum-free medium or low-serum medium, and a medium additive and a medium composition to be used for the method. Furthermore, the present invention aims to provide a method for producing a cell population with an expanded number of desired cells, particularly immunocompetent cells, in a serum-free medium or low-serum medium, and a medicament containing a cell population obtained by said method.

### [Solution to Problem]

In view of the above-mentioned problems, the present inventor prepared various factors to be added to a serum-free or low-serum medium and investigated their effects on cell culture or proliferation. As a result, an effect of maintaining and promoting cell proliferation comparable to that achieved by culture in a serum-containing medium was confirmed even when a serum-free medium or a low-serum medium was used, by adding imidazole dipeptide or a salt thereof (e.g., carnosine), taurine or a precursor thereof or a salt thereof (e.g., taurine), and lysophosphatidic acid or a derivative thereof (e.g., lysophosphatidylcholine (LPC)). It was also found that vitamin E (e.g., α-tocopherol) and/or boric acid can be further added when desired. Based on these findings, the inventor has established more preferable culture conditions and completed the present invention.

Accordingly, the present invention provides the following.
[1] A method for culturing a cell, comprising a step of culturing the cell in a serum-free medium or low-serum medium comprising (ia) imidazole dipeptide or a salt thereof, or vitamin E, (ii) taurine or a precursor thereof, or a salt thereof, and (iii) lysophosphatidic acid or a derivative thereof.
[2] The method of [1], comprising a step of culturing the cell in a serum-free medium or low-serum medium comprising (i) imidazole dipeptide or a salt thereof, (ii) taurine or a precursor thereof, or a salt thereof, and (iii) lysophosphatidic acid or a derivative thereof.
[3] The method of [1] or [2], wherein the imidazole dipeptide or a salt thereof is carnosine or anserine, or a salt thereof.
[4] The method of any of [1] to [3], wherein the imidazole dipeptide or a salt thereof is carnosine or a salt thereof.
[5] The method of any of [2] to [4], wherein the aforementioned medium further comprises vitamin E.
[6] The method of [1], comprising a step of culturing the cell in a serum-free medium or low-serum medium comprising (ib) vitamin E, (ii) taurine or a precursor thereof, or a salt thereof, and (iii) lysophosphatidic acid or a derivative thereof.
[7] The method of any of [1] to [6], wherein the lysophosphatidic acid or a derivative thereof is lysophosphatidylcholine (LPC).
[8] The method of any of [1] to [7], wherein the aforementioned medium further comprises boric acid or a salt thereof.
[9] The method of any of [1] to [8], wherein the aforementioned serum-free medium or low-serum medium is a serum-free medium.
[10] The method of any of [1] to [9], wherein the cell is an immunocompetent cell.
[11] The method of [10], wherein the immunocompetent cell is selected from dendritic cell, B cell, T cell, and natural killer cell.
[12] The method of [10] or [11], wherein the immunocompetent cell is a T cell.
[13] The method of any of [1] to [12], wherein the cell is derived from a pluripotent stem cell.
[14] The method of [13], wherein the pluripotent stem cell is an iPS cell.
[14a] The method of any of [1] to [14], wherein the cell is a cell into which a chimeric antigen receptor (CAR) has been introduced.
[14b] The method of [14a], wherein the cell is a T cell into which a chimeric antigen receptor has been introduced (CAR-T cell) or a natural killer cell into which a chimeric antigen receptor has been introduced (CAR-NK cell).
[15] A method for proliferating a cell, comprising a step of culturing the cell in a serum-free medium or low-serum medium comprising (ia) imidazole dipeptide or a salt thereof, or vitamin E, (ii) taurine or a precursor thereof, or a salt thereof, and (iii) lysophosphatidic acid or a derivative thereof.
[16] The method of [15], comprising a step of culturing the cell in a serum-free medium or low-serum medium comprising (i) imidazole dipeptide or a salt thereof, (ii) taurine or a precursor thereof, or a salt thereof, and (iii) lysophosphatidic acid or a derivative thereof.
[17] The method of [15] or [16], wherein the imidazole dipeptide or a salt thereof is carnosine or anserine, or a salt thereof.
[18] The method of any of [15] to [17], wherein the imidazole dipeptide or a salt thereof is carnosine or a salt thereof.
[19] The method of any of [16] to [18], wherein the aforementioned medium further comprises vitamin E.
[20] The method of [15], comprising a step of culturing the cell in a serum-free medium or low-serum medium comprising (ib) vitamin E, (ii) taurine or a precursor thereof, or a salt thereof, and (iii) lysophosphatidic acid or a derivative thereof.
[21] The method of any of [15] to [20], wherein the lysophosphatidic acid or a derivative thereof is lysophosphatidylcholine (LPC).
[22] The method of any of [15] to [21], wherein the aforementioned medium further comprises boric acid or a salt thereof.
[23] The method of any of [15] to [22], wherein the aforementioned serum-free medium or low-serum medium is a serum-free medium.
[24] A method for producing a cell population in which the number of desired cells is expanded, comprising a step of culturing a cell population comprising the desired cells in a serum-free medium or low-serum medium comprising (ia) imidazole dipeptide or a salt thereof, or vitamin E, (ii) taurine or a precursor thereof, or a salt thereof, and (iii) lysophosphatidic acid or a derivative thereof.
[25] The method of [24], comprising a step of culturing the cell population comprising the desired cells in a serum-free medium or low-serum medium comprising (i) imidazole dipeptide or a salt thereof, (ii) taurine or a precursor thereof, or a salt thereof, and (iii) lysophosphatidic acid or a derivative thereof.
[26] The method of [24] or [25], wherein the imidazole dipeptide or a salt thereof is carnosine or anserine, or a salt thereof.
[27] The method of any of [24] to [26], wherein the imidazole dipeptide or a salt thereof is carnosine or a salt thereof.
[28] The method of any of [25] to [27], wherein the aforementioned medium further comprises vitamin E.
[29] The method of [24], comprising a step of culturing the cell population comprising the desired cells in a serum-free medium or low-serum medium comprising (ib) vitamin E, (ii) taurine or a precursor thereof, or a salt thereof, and (iii) lysophosphatidic acid or a derivative thereof.
[30] The method of any of [24] to [29], wherein the lysophosphatidic acid or a derivative thereof is lysophosphatidylcholine (LPC).
[31] The method of any of [24] to [30], wherein the aforementioned medium further comprises boric acid or a salt thereof.
[32] The method of any of [24] to [31], wherein the aforementioned serum-free medium or low-serum medium is a serum-free medium.
[33] A serum-free or low-serum medium composition comprising (ia) imidazole dipeptide or a salt thereof, or vitamin E, (ii) taurine or a precursor thereof, or a salt thereof, and (iii) lysophosphatidic acid or a derivative thereof.
[34] The composition of [33], comprising (i) imidazole dipeptide or a salt thereof, (ii) taurine or a precursor thereof, or a salt thereof, and (iii) lysophosphatidic acid or a derivative thereof.
[35] The composition of [33], comprising (ib) vitamin E, (ii) taurine or a precursor thereof, or a salt thereof, and (iii) lysophosphatidic acid or a derivative thereof.
[36] A medium additive comprising (ia) imidazole dipeptide or a salt thereof, or vitamin E, (ii) taurine or a precursor thereof, or a salt thereof, and (iii) lysophosphatidic acid or a derivative thereof, wherein the medium is a serum-free medium or a low-serum medium.
[37] A medium additive comprising (i) imidazole dipeptide or a salt thereof, (ii) taurine or a precursor thereof, or a salt thereof, and (iii) lysophosphatidic acid or a derivative thereof, wherein the medium is a serum-free medium or a low-serum medium.
[38] The medium additive of [36] or [37], wherein the imidazole dipeptide or a salt thereof is carnosine or anserine, or a salt thereof.
[39] The medium additive of any of [36] to [38], wherein the imidazole dipeptide or a salt thereof is carnosine or a salt thereof.
[40] The medium additive of any of [37] to [39], wherein the aforementioned medium further comprises vitamin E.
[41] A medium additive comprising (ib) vitamin E, (ii) taurine or a precursor thereof, or a salt thereof, and (iii) lysophosphatidic acid or a derivative thereof, wherein the medium is a serum-free medium or a low-serum medium.
[42] The medium additive of any of [36] to [41], wherein the lysophosphatidic acid or a derivative thereof is lysophosphatidylcholine (LPC).
[43] The medium additive of any of [36] to [42], wherein the aforementioned medium further comprises boric acid or a salt thereof.
[44] The medium additive of any of [36] to [43], wherein the aforementioned serum-free medium or low-serum medium is a serum-free medium.
[45] A medicament comprising a cell population obtained by the method of any of [24] to [32].
[46] The medicament of [45], which is for use in the prophylaxis and/or treatment of cancer.
[47] A method for the prophylaxis and/or treatment of cancer, comprising administering a cell population obtained by the method of any of [24] to [32] to a subject in need thereof.
[48] A cell population obtained by the method of any of [24] to [32], which is for use in the prophylaxis and/or treatment of cancer.
[49] Use of a cell population obtained by the method of any of [24] to [32] in the manufacture of a medicament for the prophylaxis and/or treatment of cancer.

### [Advantageous Effects of Invention]

According to the present invention, a cell proliferation maintaining-promoting effect comparable to that by culture in a serum-containing medium can be obtained even when a serum-free medium or low-serum medium is used. In particular, culture in a serum-free medium or low-serum medium that does not require extensive washing is preferred for cells that are vulnerable to a stress caused by operations such as washing of cell and the like, and the cells are suitable for culture under such conditions.

### (DETAILED DESCRIPTION OF THE INVENTION)

The present invention is described in the following. The terms used in the present specification have the meanings generally used in the pertinent field unless otherwise specified.

### (1) Cell culture method (hereinafter also to be referred to as "the culture method of the present invention"), cell proliferation method (hereinafter also to be referred to as "the proliferation method of the present invention")

The culture method or proliferation method of the present invention contains a step of culturing cells in a serum-free medium or low-serum medium containing (i) imidazole dipeptide or a salt thereof, (ii) taurine or a precursor thereof or a salt thereof, and (iii) lysophosphatidic acid or a derivative thereof. The serum-free medium or low-serum medium may contain (iv) vitamin E and/or (v) boric acid or a salt thereof when desired.

In the culture method or proliferation method of the present invention, vitamin E may also be used when desired, in addition to or instead of (i) imidazole dipeptide or a salt thereof. In this case, component (iv) is not necessary.

Therefore, the culture method or proliferation method of the present invention may be the method containing a step of culturing cells in a serum-free medium or low-serum medium containing (ia) imidazole dipeptide or a salt thereof, or vitamin E, (ii) taurine or a precursor thereof, or a salt thereof, and (iii) lysophosphatidic acid or a derivative thereof.

### 1-1. Cell

The type of cell to be the target of culture in the culture method and proliferation method of the present invention is not particularly limited. Examples of the cell type include reproductive cells such as sperm and eggs, somatic cells that compose living organisms, stem cells (pluripotent stem cells, etc.) and cells induced to differentiate from stem cells, progenitor cells, cancer cells isolated from living organisms, cells (cell lines) isolated from living organisms that have acquired immortalization ability and are stably maintained outside the body, cells isolated from living organisms that have been artificially modified genetically, and cells isolated from living organisms and having artificially-exchanged nucleus. Examples of the somatic cells that compose living organisms and the cells induced to differentiate from stem cells include, but are not limited to, immunocompetent cells (natural killer (NK) cells, macrophages, monocytes, mast cells, dendritic cells, Langerhans cells, neutrophils, eosinophils, basophils, B cells, T cells, etc.), fibroblasts, bone marrow cells, red blood cells, platelets, bone cells, pericytes, keratinocytes, adipocytes, mesenchymal cells, epithelial cells, epidermal cells, endothelial cells, vascular endothelial cells, hepatic parenchymal cells, chondrocytes, cumulus cells, nervous system cells, glial cells, neurons, oligodendrocytes, microglia, astrocytes, cardiac cells, esophageal cells, muscle cells (e.g., smooth muscle cells or skeletal muscle cells), pancreatic beta cells, melanocytes, hematopoietic progenitor cells (e.g., CD34-positive cells derived from umbilical cord blood), and mononuclear cells. Somatic cells include cells obtained from any tissue, such as skin, kidney, spleen, adrenal gland, liver, lung, ovary, pancreas, uterus, stomach, colon, small intestine, large intestine, bladder, prostate, testes, thymus, muscle, connective tissue, bone, cartilage, vascular tissue, blood (including umbilical cord blood), bone marrow, heart, eye, brain, or neural tissue.

Stem cell is a cell that has the ability to replicate itself and to differentiate into other multiple lineages of cells. Examples thereof include, but are not limited to, embryonic tumor cells, pluripotent stem cells, neural stem cells, hematopoietic stem cells, mesenchymal stem cells, liver stem cells, pancreatic stem cells, muscle stem cells, germline stem cells, intestinal stem cells, cancer stem cells, and hair follicle stem cells.

"Pluripotent stem cells" refer to cells that have the ability to self-replicate and differentiate/proliferate, and have the ability to differentiate into all tissues and cells that constitute a living organism. Examples of pluripotent stem cells include embryonic stem cells (ES cells), embryonic germ cells (EG cells), induced pluripotent stem cells (iPS cells), and pluripotent stem cells induced and selected by stress or cell stimulation. Stem cells established by culturing early embryos produced by nuclear transplantation of the nucleus of a somatic cell are also preferred as pluripotent stem cells (Nature, 385, 810 (1997); Science, 280, 1256 (1998); Nature Biotechnology, 17, 456 (1999); Nature, 394, 369 (1998); Nature Genetics, 22, 127 (1999); Proc. Natl. Acad. Sci. USA, 96, 14984 (1999); Nature Genetics, 24, 109 (2000)). In the present invention, iPS cells are preferred as pluripotent stem cells. iPS cells can be identified using undifferentiated markers due to the undifferentiated nature of iPS cells as indicators. Examples of undifferentiated markers include alkaline phosphatase, Oct3/4, Sox2, Nanog, ERas, Esgl, etc. The methods for detecting these undifferentiated markers include methods for detecting mRNA (using primers and probes), immunological detection methods (using antibodies and labels), and the like.

A cell induced to differentiate from a stem cell (differentiation-induced cell) is any cell that has been subjected to a differentiation-inducing treatment that induces differentiation from a stem cell into a specific type of cell.

A cell line is a cell that has acquired the ability to proliferate indefinitely through artificial manipulation outside of a living organism. Examples thereof include, but are not limited to, CHO (Chinese hamster ovary cell line), HCT116, Huh7, HEK293 (human embryonic kidney cell), HeLa (human uterine cancer cell line), HepG2 (human hepatoma cell line), UT7/TPO (human leukemia cell line), MDCK, MDBK, BHK, C-33A, HT-29, AE-1, 3D9, Ns0/1, Jurkat, NIH3T3, PC12, S2, Sf9, Sf21, High Five (trade name), Vero and the like.

In one preferred embodiment, the cell to be the target of culture is an immunocompetent cell. The immunocompetent cells refer to cells involved in various immune responses in vivo. Examples of immunocompetent cells include natural killer (NK) cells, macrophages, monocytes, mast cells, dendritic cells, Langerhans cells, neutrophils, eosinophils, basophils, B cells, and T cells, and are preferably selected from dendritic cells, B cells, T cells, and natural killer (NK) cells. More preferably, the immunocompetent cells are T cells.

T cells include CD4 positive CD8 negative T cells, CD4 negative CD8 positive T cells, αβ-T cells, γδ-T cells, regulatory T cells, and NKT cells. T cells may be subsets such as naive T cells, effector T cells, memory T cells, or the like.

The immunocompetent cells may be cells isolated from humans (primary cells) or cells obtained by differentiation from stem cells such as pluripotent stem cells (e.g., iPS cells, ES cells), hematopoietic stem cells, and mesenchymal stem cells, and are preferably primary cells and cells obtained by differentiation from pluripotent stem cells (particularly iPS cells). As the primary cells, primary T cells derived from human are preferred. Differentiation induction from stem cells to immunocompetent cells can be performed by a method known in the art, depending on the type of stem cells used and the type of immunocompetent cells of interest. In one embodiment, differentiation induction from iPS cells to T cells and differentiation induction from iPS cells to NK cells can be performed by the methods described in the Examples.

In addition, the cells to be the target may be either autologous cells or allogeneic cells. In the present invention, the "autologous cells " refers to cells obtained from a subject receiving a cell population (described below) produced by the culture method of the present invention or the proliferation method of the present invention, or cells derived from the obtained cells, and "allogeneic cells" refers to cells that are not the above-mentioned "autologous cells".

In the present invention, the cell to be the target of culture may be a cell that has been artificially modified genetically. Examples of such cells include immunocompetent cells into which a CAR gene has been introduced (T cells into which a CAR gene has been introduced and natural killer cells into which a CAR gene has been introduced), T cells into which an exogenous T cell receptor (TCR) has been introduced, and immunocompetent cells into which genes for expressing cytokines and/or chemokines have been introduced.

CAR is a structure including, from the N-terminal side to the C-terminal side of a protein, an extracellular domain specific to a target, a transmembrane domain, and an intracellular signal domain for the effector function of immune cells, and the CAR gene is a gene encoding this receptor. The extracellular domain contains an antigen recognition site that exhibits specific binding property to a target. The transmembrane domain is present between the extracellular domain and the intracellular signal domain. The intracellular signal domain transmits a signal necessary for immune cells to exert the effector functions thereof. That is, an intracellular signal domain capable of transmitting a signal necessary for activating immune cells when the extracellular domain binds to a target antigen is used. There have been several reports of experiments, clinical studies, and the like using CARs (e.g., Rossig C, et al. Mol Ther 10:5-18, 2004; Dotti G, et al. Hum gene Ther 20:1229-1239, 2009; Ngo MC, et al. Hum Mol Genet 20 (R1):R93-99, 2011; Ahmed N, et al. Mol Ther 17:1779-1787, 2009; Pule MA, et al. Nat Med 14:1264-1270, 2008; Louis CU, et al. Blood 118:6050-6056, 2011; Kochenderfer JN, et al. Blood 116:4099-4102, 2010; Kochenderfer JN, et al. Blood 119 :2709-2720, 2012; Porter DL, et al. N Engl J Med 365:725-733, 2011; Kalos M, et al. Sci Transl Med 3:95ra73,2011; Brentjens RJ, et al. Blood 118:4817-4828, 2011; Brentjens RJ, et al. Sci Transl Med 5:177 ra38, 2013), and the CAR in the present invention can be constructed by referring to these reports.

Preferred one embodiment of cells to which the culture method and proliferation method of the present invention are applied includes T cells into which a CAR gene has been introduced (CAR-T cells) and NK cells into which a CAR gene has been introduced (CAR-NK cells), more preferably CAR-T cells and CAR-NK cells derived from pluripotent stem cells, further preferably CAR-T cells and CAR-NK cells derived from iPS cells, and further more preferably CAR-T cells derived from iPS cells. CAR-T cells and CAR-NK cells can be obtained by introducing a CAR gene into T cells and NK cells, or their precursor cells such as pluripotent stem cells, respectively. For example, the CAR-T cells may be cells obtained by introducing a CAR gene into cells obtained by differentiating cells such as iPS cells, ES cells, hematopoietic stem cells, and mesenchymal stem cells into T cells, or cells obtained by differentiating cells such as iPS cells, ES cells, hematopoietic stem cells, and mesenchymal stem cells, into which a CAR gene has been introduced, into T cells. Preferably, the CAR-T cells are derived from iPS cells and obtained by differentiating iPS cells, into which a CAR gene has been introduced, into T cells, or the CAR-T cells are derived from iPS cells and obtained by introducing a CAR gene into T cells obtained by differentiating iPS cells, and these are sometimes collectively referred to as "iPS cell-derived CAR-T cells". More preferably, the CAR-T cells are derived from iPS cells and obtained by introducing a CAR gene into T cells obtained by differentiating iPS cells. For example, in the case of CAR-NK cells, the CAR-NK cells may be cells obtained by introducing a CAR gene into cells obtained by differentiating cells such as iPS cells, ES cells, hematopoietic stem cells, and mesenchymal stem cells into NK cells, or cells obtained by differentiating cells such as iPS cells, ES cells, hematopoietic stem cells, and mesenchymal stem cells, into which a CAR gene has been introduced, into NK cells. Preferably, the CAR-NK cells are derived from iPS cells and obtained by differentiating iPS cells, into which a CAR gene has been introduced, into NK cells, or the CAR-NK cells are derived from iPS cells and obtained by introducing a CAR gene into NK cells obtained by differentiating iPS cells, and these are sometimes collectively referred to as "iPS cell-derived CAR-NK cells". More preferably, the CAR-NK cells are derived from iPS cells and obtained by introducing a CAR gene into NK cells obtained by differentiating iPS cells.

The induction of differentiation from iPS cells (into which a CAR gene may be introduced, when desired) into T cells, or the induction of differentiation from iPS cells (into which a CAR gene may be introduced, when desired) into NK cells, can be performed using a known method, specifically, by the method described in the Examples.

The CAR gene is generally introduced into cells using a CAR expression vector. The CAR expression vector means a nucleic acid molecule capable of transporting a nucleic acid molecule encoding a CAR gene into cells. It may be DNA or RNA and is not particularly limited as to its form or origin, and various types of vectors may be used. The vector may be a viral vector or a non-viral vector. Examples of viral vectors include retroviral vectors, lentiviral vectors, adenoviral vectors, adeno-associated viral vectors, herpes viral vectors, Sendai viral vectors, and vaccinia viral vectors. Among these, in retroviral vectors, lentiviral vectors, and adeno-associated viral vectors, the target gene incorporated into the vector is incorporated into the host chromosome, and stable and long-term expression can be expected. Each viral vector can be produced according to a conventional method or using a commercially available dedicated kit. Examples of non-viral vectors include plasmid vectors, liposome vectors, positively charged liposome vectors (Felgner, P.L., Gadek, T.R., Holm, M. et al., Proc. Natl. Acad. Sci., 84:7413-7417, 1987), YAC vectors, BAC vectors, and artificial chromosome vectors.

In one embodiment, the CAR gene can be introduced into cells by the method described in the Examples.

### 1-2. Medium/medium composition

The medium used in the culture method and proliferation method of the present invention is a serum-free medium or low-serum medium containing (i) imidazole dipeptide or a salt thereof, (ii) taurine or a precursor thereof or a salt thereof, and (iii) lysophosphatidic acid or a derivative thereof, and further containing (iv) vitamin E and/or (v) boric acid or a salt thereof when desired (hereinafter sometimes collectively referred to as "the medium of the present invention").

In the medium of the present invention, vitamin E may also be used when desired, in addition to or instead of (i) imidazole dipeptide or a salt thereof. When vitamin E is used, component (iv) does not need to be further added.

Therefore, the medium used in the culture method and proliferation method of the present invention bay be a serum-free medium or low-serum medium containing containing (ia) imidazole dipeptide or a salt thereof, or vitamin E, (ii) taurine or a precursor thereof, or a salt thereof, and (iii) lysophosphatidic acid or a derivative thereof.

The present invention can provide a serum-free or low-serum medium composition containing a basal medium (described below) to which the above-mentioned components (ia) to (iii).

The present invention can provide a serum-free or low-serum medium composition containing a basal medium (described below) to which the above-mentioned components (i) to (iii) and, when desired, components (iv) and/or (v) are further added.

### (i) Imidazole dipeptide or a salt thereof (hereinafter also collectively referred to as imidazole dipeptides)

Imidazole dipeptide is one type of peptide in which two amino acids are bonded via an imidazole ring. Examples of imidazole dipeptides include carnosine consisting of β-alanine and L-histidine, anserine consisting of β-alanine and 1-methyl-L-histidine, balenine consisting of valine and L-histidine, and homocarnosine having a structure similar to carnosine. Other examples include dipeptide consisting of alanine and L-histidine, dipeptide consisting of glycine and L-histidine, and dipeptide consisting of lysine and L-histidine. Preferred are carnosine or anserine, or a salt thereof. Salts of imidazole dipeptides are not particularly limited as long as they are pharmaceutically acceptable, and include inorganic acid salts such as hydrochloride, sulfate, phosphate, diphosphate, hydrobromide, and nitrate; and organic acid salts such as acetate, maleate, fumarate, tartrate, succinate, citrate, lactate, methanesulfonate, p-toluenesulfonate, salicylate, oxalate, stearate, ascorbate, malate, adipate, and gluconate. For example, salts of anserine include hydrochloride, nitrate, and acetate, and salts of carnosine include hydrochloride, nitrate, and acetate.

Preferred imidazole dipeptides in the present invention are carnosine, anserine, and salts thereof, more preferably carnosine or a salt thereof.

Imidazole dipeptides are contained in the meat of fish, shellfish, livestock, poultry, whales, and the like, and can be prepared from these as raw materials according to conventionally-known extraction methods and purification methods. For convenience, commercially available imidazole dipeptides can also be used. In the present invention, the origin of the imidazole dipeptide used does not matter, and one type of the the aforementioned imidazole dipeptide and/or a salt thereof may be each used alone or in combination of two or more types thereof.

The concentration of imidazole dipeptides in the medium is not particularly limited as long as the desired effect is obtained, and may be increased or decreased as appropriate depending on the type of imidazole dipeptide or a salt thereof to be used. It is generally 1 to 50 mM, preferably 3 to 40 mM, more preferably 5 to 30 mM, particularly preferably 10 to 25 mM. When two or more types are used in combination, it is the total concentration thereof. In the present specification, the concentration of imidazole dipeptide or a salt thereof is, unless otherwise specified, the concentration converted to carnosine, which is a representative imidazole dipeptide. When the concentration is too high, it affects the cell survival rate, and when it is too low, the effect of promoting cell proliferation is weak.

### (ii) Taurine or a precursor thereof or a salt thereof (hereinafter also collectively referred to as taurines)

Taurine is an organic sulfur compound and one type of non-essential amino acid synthesized from cysteine or methionine. The taurine precursor is a substance that is directly or indirectly converted into taurine, and is not particularly limited as long as the desired effect is obtained when cells are cultured in the presence of the taurine precursor. For example, the taurine precursor may be one or more selected from the group consisting of cysteine sulfinic acid, cysteamine, cysteic acid, and hypotaurine. The salt of taurine or a precursor thereof is not particularly limited as long as it is pharmaceutically acceptable, and includes alkali metal salts such as sodium salts and potassium salts; alkaline earth metal salts such as calcium salts and magnesium salts; aluminum salts; and a salt with an organic amine such as triethylamine, triethanolamine, morpholine, piperazine, and pyrrolidine.

In the present invention, preferred taurines are taurine, hypotaurine and salts thereof, more preferably taurine or a salt thereof.

Taurines are contained in the meat of fish, shellfish, livestock and the like, and thus livestock-derived taurine and marine product-derived taurine can be used, which are obtained using the meat as a raw material and conventionally known extraction and purification methods. For convenience, commercially available synthetic taurine of reagent/pharmaceutical grade can also be used.

The concentration of taurines in the medium is not particularly limited as long as the desired effect is obtained, and may be increased or decreased as appropriate depending on the type of taurines to be used. It is generally 0.1 to 20 mM, preferably 0.5 to 15 mM, further preferably 1 to 10 mM, particularly preferably 2 to 5 mM. In the present specification, the concentration of taurines is, unless otherwise specified, the concentration converted to taurine. When the concentration is too high, it affects the cell survival rate, and when it is too low, the effect of promoting cell proliferation is weak.

### (iii) Lysophosphatidic acid or a derivative thereof (hereinafter also collectively referred to as lysophosphatidic acids)

Lysophosphatidic acid (LPA) is a phospholipid derivative that has a structure in which the acyl group has been removed from phosphatidic acid and can transmit various signals into cells. It is known that LPA exhibits a variety of pharmacological actions through several G protein-coupled receptors on the cell surface. There are eight LPA receptor subtypes with different roles, and they are called LPA1, LPA2, LPA3, LPA4, LPA5, LPA6, GPR87, and GPR35. Examples of lysophosphatidic acid derivatives include lysophosphatidylserine (LPS), which is a serine-based lysophosphatidic acid, lysophosphatidylethanolamine (LPE), which is an ethanolamine-based lysophosphatidic acid, lysophosphatidylcholine (LPC), which is a choline-based lysophosphatidic acid, and lysophosphatidylinositol, which is an inositol-based lysophosphatidic acid.

In the present invention, the preferred one of lysophosphatidic acids is lysophosphatidylcholine.

Lysophosphatidic acid or a derivative thereof may exist as a salt thereof. The salt is not particularly limited as long as it is pharmaceutically acceptable, and examples thereof include alkali metal salts such as sodium salt and potassium salt; alkaline earth metal salts such as calcium salt and magnesium salt; aluminum salt; and a salt with an organic amine such as triethylamine, triethanolamine, morpholine, piperazine, and pyrrolidine.

The production method of lysophosphatidic acids is not particularly limited, and those produced by known methods can be used. A specific production method is to produce lysophosphatidic acids by enzymatic lyso-formation of lecithin containing phosphatidic acid. For convenience, commercially available lysophosphatidic acids can also be used.

The concentration of lysophosphatidic acids in the medium is not particularly limited as long as the desired effect is obtained, and may be increased or decreased as appropriate depending on the type of compound to be used. It is generally 0.1 to 50 mg/L, preferably 0.5 to 25 mg/L, further preferably 1 to 15 mg/L, particularly preferably 2 to 10 mg/L. In the present specification, the concentration of lysophosphatidic acid or a derivative thereof is, unless otherwise specified, the concentration converted to lysophosphatidylcholine. When the concentration is too high, it affects the cell survival rate, and when it is too low, the effect of promoting cell proliferation is weak.

### (iv) Vitamin E

Vitamin E is a fat-soluble antioxidant vitamin, and is mainly classified into two main groups, tocopherols and tocotrienols. These compounds have a common feature of having a phenol ring and a long fatty chain bonded thereto. Tocopherols have four isomers (α, β, γ, δ) that differ depending on the position of the methyl group, and tocotrienols also have similar isomers. These may be each used alone or in combination of two or more types thereof.

In the present invention, preferred vitamin E is tocopherol or a salt thereof, more preferably α-tocopherol or a salt thereof.

Vitamin E may be in the form of a salt. The salt of vitamin E is not particularly limited as long as it is pharmaceutically acceptable. For example, it may be a salt with an organic base (e.g., a salt with an organic amine such as methylamine, triethylamine, triethanolamine, morpholine, piperazine, pyrrolidine, tripyridine, picoline, etc.), or a salt with an inorganic base (e.g., an ammonium salt; an alkali metal salt such as sodium salt or potassium salt; an alkaline earth metal salt such as calcium salt or magnesium salt; or a metal salt such as aluminum salt). Furthermore, vitamin E may be in the form of a derivative. Examples of vitamin E derivatives include vitamin E acetate (tocopherol acetate), vitamin E nicotinate, vitamin E succinate, and vitamin E linoleate. The aforementioned vitamin E (including salts and derivatives) may be a commercially available product.

The concentration of vitamin E in the medium is not particularly limited as long as the desired effect is obtained, and may be increased or decreased as appropriate depending on the type of compound to be used. It is generally 10 to 1000 µM, preferably 20 to 500 µM, further preferably 40 to 300 µM, particularly preferably 100 to 250 µM. In the present specification, the concentration of vitamin E is, unless otherwise specified, the concentration converted to α-tocopherol. When the concentration is too high, it affects the cell survival rate, and when it is too low, the effect of promoting cell proliferation is weak.

### (v) Boric acid or a salt thereof

In the present specification, boric acid is not particularly limited as long as it is pharmaceutically acceptable, and examples thereof include orthoboric acid, metaboric acid, and tetraboric acid. Among these boric acids, orthoboric acid is preferably used. Salts of boric acid are not particularly limited as long as they are pharmaceutically acceptable, and examples thereof include alkali metal salts such as sodium salts and potassium salts; alkaline earth metal salts such as calcium salts and magnesium salts; aluminum salts; and a salt with an organic amine such as triethylamine, triethanolamine, morpholine, piperazine, and pyrrolidine. In addition, boric acid or a salt thereof may be in the form of a hydrate, such as borax.

The aforementioned boric acid (including salts) may be a commercially available product.

The concentration of boric acid or a salt thereof in the medium is not particularly limited as long as the desired effect is obtained. It is generally 0.1 to 0.9 mM, preferably 0.2 to 0.8 mM, further preferably 0.2 to 0.7 mM, particularly preferably 0.3 to 0.6 mM. In the present specification, the concentration of boric acid or a salt thereof is, unless otherwise specified, the concentration converted to boric acid. When the concentration is too high, it affects the cell survival rate, and when it is too low, the effect of promoting cell proliferation is weak.

In the present specification, the "serum-free medium" means a medium that is substantially free of, preferably free of, unconditioned or unpurified serum, and a medium that is contaminated with purified blood-derived components or animal tissue-derived components (e.g., growth factors) is considered to be serum-free medium. As a medium, a medium used for culturing animal cells (for convenience, hereinafter also referred to as a basal medium) or a medium for perfusion culture is generally used. Here, "substantially free of" means that it does not contain at all, or even if it contains, it is below the detection limit.

Examples of basal media include, but are not limited to, BME medium, BGJb medium, CMRL 1066 medium, Glasgow MEM medium, Improved MEM ZincOption medium, IMDM medium, Medium 199 medium, Eagle MEM medium, αMEM medium, DMEM medium, Ham's medium, RPMI 1640 medium, Fischer's medium, F12 medium, and mixed media thereof (e.g., Advanced DMEM/F12 medium, etc.).

Perfusion culture is a culture method in which a culture system containing cells is continuously supplied with the medium while an equal amount of cell-free culture supernatant is continuously removed from the culture system to maintain the culture system in a steady state. Because perfusion culture requires a higher cell density than general culture methods, the medium for perfusion culture generally has a higher concentration of nutrient components than basal medium.

These media can be purchased from Invitrogen, SIGMA, FUJIFILM Wako Pure Chemical Corporation, Sumitomo Pharma, etc., and media with the same name or the same product name have the same composition regardless of manufacturer.

In the present specification, the "low-serum medium" refers to a medium in which serum has been added to a basal medium and characterized in that the serum concentration is reduced compared to serum-containing media used generally (serum concentration: 5-20% (serum concentration is expressed in v/v% in the present specification). The serum concentration of the low-serum medium may be less than 5%, less than 2%, less than 1%, less than 0.1%, less than 0.01%, or less than 0.001%, but is generally 0.1% or more, or 1% or more. Specifically, the aforementioned serum concentration is preferably 0.1% or more and less than 5%, more preferably 1% or more and less than 4%.

The medium used in the culture method and proliferation method of the present invention may also contain a serum replacement. Examples of the serum replacement include albumin (e.g., lipid-rich albumin), transferrin, fatty acids, collagen precursors, insulin, trace elements (e.g., zinc, selenium, etc.), ITS supplement (mixture of insulin, transferrin, and selenite), B-27 supplement, N2 supplement, knockout serum replacement, 2-mercaptoethanol or 3'-thiolglycerol, or equivalents thereof. These serum replacements are also commercially available. Knockout serum replacement can be purchased from Invitrogen. Other serum replacements can be purchased from Invitrogen, SIGMA, FUJIFILM Wako Pure Chemical Corporation, Sumitomo Pharma, and the like, and the composition of reagents or additives with the same name or trade name is equivalent regardless of the manufacturer.

The medium of the present invention may further contain, in addition to the basal medium, components that are favorable for cell proliferation. Examples of such components include sugars such as glucose, fructose, sucrose, and maltose; amino acids or amino acid derivatives such as glycine, serine, alanine, valine, leucine, isoleucine, methionine, phenylalanine, tyrosine, tryptophan, proline, threonine, cysteine, asparagine, glutamine, aspartic acid, glutamic acid, arginine, lysine, histidine, ornithine, theanine, citrulline, betaine, carnitine, creatine, and pantothenic acid; proteins such as albumin and transferrin; peptides such as glycylglycylglycine and soybean peptide; serum; choline, vitamins such as vitamin B group (thiamine, riboflavin, pyridoxine, cyanocobalamin, biotin, folic acid, pantothenic acid, nicotinamide, etc.), vitamin C (ascorbic acid), and vitamin C derivatives; fatty acids such as oleic acid, arachidonic acid, and linoleic acid; lipids such as cholesterol; nucleosides such as adenosine, thymidine, guanosine, cytidine, uridine, and inosine; inosinic acid; hypoxanthine; inorganic salts such as sodium chloride, potassium chloride, calcium chloride, magnesium sulfate, and sodium dihydrogen phosphate; trace elements such as zinc, copper, selenium, vanadium, manganese, nickel, silicon, tin, molybdenum, cadmium, chromium, silver, aluminum, barium, cobalt, germanium, iodine, bromine, fluorine, rubidium, and zirconium; buffering agents such as N,N-bis(2-hydroxyethyl)-2-aminoethanesulfonic acid (BES), 4-(2-hydroxyethyl)-1-piperazineethanesulfonic acid (HEPES), and N-[tris(hydroxymethyl)methyl]glycine (Tricine); antibiotics such as amphotericin B, kanamycin, gentamicin, and streptomycin; cell adhesion factors and extracellular matrix components such as Type I collagen, Type II collagen, fibronectin, laminin, poly-L-lysine, and poly-D-lysine; cytokines and growth factors such as interleukins, fibroblast growth factors (FGF), hepatocyte growth factors (HGF), transforming growth factors (TGF)-α, transforming growth factors (TGF)-β, vascular endothelial growth factors (VEGF), and activin A; and hormones such as dexamethasone, hydrocortisone, estradiol, progesterone, glucagon, and insulin. Appropriate components can be selected and used at appropriate concentrations according to the type of cells to be cultured.

The medium may further contain an additive factor to support cell survival and proliferation. The additive factor may be a type 1 cytokine family member, a type 2 cytokine family member, a TNF superfamily cytokine, an IL-1 family cytokine, or other cytokines (TNF-β, etc.), specifically IL-1 to IL-41, and the like, preferably IL-1, IL-2, IL-7, IL-15, IL-18, IL-21, and the like. The additive factor may be prepared according to a conventional method, or a commercially available product may be used. The additive factor may be from an animal species other than human, but is preferably from human origin (which may be recombinant).

In the present specification, "xeno-free" refers to conditions under which components derived from organisms other than the organism of the cells to be cultured are excluded.

### 1-3. Cell culture and Cell proliferation

The present invention also provides a method for culturing cells (the culture method of the present invention) and a method for proliferating cells (the proliferation method of the present invention), characterized in that the cells are cultured in the medium of the present invention (described above) (the culture method of the present invention).

The incubator to be used for culturing the cells is not particularly limited as long as it is capable of culturing cells, and examples of the incubator include flasks, tissue culture flasks, dishes, Petri dishes, tissue culture dishes, multi-dishes, microplates, microwell plates, multi-plates, multi-well plates, microslides, chamber slides, petri dishes, tubes, trays, culture bags, roller bottles, and bioreactors. An appropriate incubator is selected according to the target cells.

The incubator may be either cell-adhesive or cell-non-adhesive, and is appropriately selected according to the purpose. A cell-adhesive incubator may be coated with any cell-supporting substrate, such as an extracellular matrix (ECM), in order to improve adhesion of the surface of the incubator to cells. The cell-supporting substrate may be any substance intended for cell adhesion.

Other culture conditions may be appropriately set. For example, the culture temperature is not particularly limited and may be about 30 to 40°C, preferably about 37°C. The CO₂ concentration may be about 1 to 10%, preferably about 2 to 5%. The oxygen partial pressure may be about 1 to 21%.

The frequency of medium exchange and the culture period in cell culture are generally determined by comprehensively considering various conditions such as cell density, culture method (adhesive culture/suspension culture), type of cell to be cultured, medium composition, culture conditions (temperature, gas concentration), amount of medium to be exchanged (total amount/partial amount), cost of medium, and lifestyle of the operator. Medium exchange is generally performed once every 2 to 3 days, once a day, or multiple times a day (e.g., twice a day), and the medium exchange can be performed at such frequencies in the culture method and proliferation method of the present invention. Furthermore, medium exchange can be performed continuously by perfusion culture. The culture period is generally about 2 days to 4 weeks, preferably about 3 days to 3 weeks, more preferably about 3 days to 2 weeks.

In the present specification, "cell proliferation" refers to a phenomenon in which cells divide repeatedly to increase their number. It includes the process in which cells form new cells through the cell cycle, and is achieved by cell division in an appropriate culture environment. Indicators of proliferation include an increase in cell number, an increase in DNA content, or the expression of a specific proliferation-related marker. Specifically, it refers to an increase in the number of cells compared to a control such as before proliferation (i.e., before the start of culture), and means at least 10%, 20%, 30%, 40%, 50%, 60%, 70%, 80%, 90%, 100%, 120%, 150%, 200%, 300%, 400%, 500%, 600%, 700%, 800%, 900%, or 1000% increase compared to the control. In particular embodiments of the present invention, the number of cells is increased by at least 10%, 20%, 30%, 40%, 50%, 60%, 70%, 80%, 90%, 100%, 120%, 150%, 200%, 300%, 400%, 500%, 600%, 700%, 800%, 900%, or 1000% compared to the number of cells of the control.

In one embodiment of the present invention, the cells to be cultured in a serum-free medium or low-serum medium containing (i) imidazole dipeptide or a salt thereof, (ii) taurine or a precursor thereof, or a salt thereof, and (iii) lysophosphatidic acid or a derivative thereof, and further containing (iv) vitamin E and/or (v) boric acid or a salt thereof when desired (i.e., the medium of the present invention) are immunocompetent cells, preferably selected from dendritic cells, B cells, T cells, and natural killer (NK) cells, more preferably T cells and NK cells, and further preferably T cells. In another embodiment of the present invention, the cells to be cultured in the medium of the present invention are primary T cells and T cells derived from pluripotent stem cells (particularly iPS cells), more preferably primary T cell-derived and pluripotent stem cell-derived (particularly iPS cell)-derived CAR-T cells, further preferably pluripotent stem cell-derived (particularly iPS cell)-derived CAR-T cells. In still another embodiment of the present invention, the cells to be cultured in the medium of the present invention are primary NK cells and pluripotent stem cell (particularly iPS cell)-derived NK cells, more preferably primary NK cell-derived and pluripotent stem cell (particularly iPS cell)-derived CAR-NK cells, further preferably pluripotent stem cell (particularly iPS cell)-derived CAR-NK cells.

In one embodiment of the present invention, culture includes an activation step and an expansion step. When cryopreserved cells are used, a recovery step may be included. In the present invention, when the culture target is a cryopreserved cell, the culture of the cell is a concept including any one, two, or all three of recovery culture consisting of a recovery step, activation culture consisting of an activation step, and expansion culture consisting of an expansion step. Preferably, the target is expansion culture. Another embodiment of the present invention is a culture of CAR-T cells and CAR-NK cells, preferably pluripotent stem cell (particularly iPS cell)-derived CAR-T cells and pluripotent stem cell (particularly iPS cell)-derived CAR-NK cells, which includes an activation step and an expansion step, and includes, where necessary, a recovery step (in the case of using cryopreserved cells, etc.).

### (Recovery step/recovery culture)

This step is performed after thawing the cryopreserved cells. Since prolonged exposure of cells to a cryoprotectant can cause damage, the cryoprotectant is promptly removed from the cells after thawing. Then, cell damage and cell dysfunction caused by freeze-thawing are restored. This step/culture method varies depending on the target cells, the cryoprotectant used, the thawing method, and the like. Most cells generally recover normally by culturing them in a medium that does not contain a cryoprotectant for several days, preferably 1 to 5 days, more preferably about 3 days.

### (Activation step/activation culture)

The method of this step is not particularly limited as long as the desired effect on the cells can be obtained, and for example, it can be performed by contacting with a stimulating substance. Generally, the cells are cultured in the presence of a stimulating substance, specifically in a medium containing the stimulating substance, for several days, preferably 1 to 5 days, more preferably about 3 days. The stimulating substance is a signal molecule that controls the activity of cells by a method such as autocrine, paracrine, or endocrine, and may be a substance that can be secreted from all cells contained in the culture system, or may be a substance that is added from the outside. Specifically, the step is carried out by contact with a substrate to which a ligand is bound, or by culturing in a medium containing a ligand, or the like.

For example, the ligand used in the activation culture of CAR-T cells, preferably pluripotent stem cell (particularly iPS cell)-derived CAR-T cells, is not particularly limited as long as it is a molecule that interacts with a surface molecule of CAR-T cells and promotes the activation thereof. Examples include CD3, which couples with TCR and is responsible for signal transduction via TCR, and molecules that specifically bind to surface molecules known as costimulatory factors for T cell activation, such as CD28, ICOS, CD137, OX40, CD27, GITR, BAFFR, TACI, BMCA, and CD40L, and have the function of transmitting an activation signal into T cells. Such molecules may be physiological ligands (or receptors) for the above-mentioned T cell surface molecules, or non-physiological ligands (or receptors) having agonistic activity. A preferred example of a non-physiological ligand is an agonist antibody.

More preferably, the T cell activation ligand to be used in the present invention is an antibody against CD3. The antibody against CD3 may be a complete antibody or a fragment thereof (e.g., Fab, F(ab')₂, Fab', scFv, Fv, reduced antibody (rIgG), dsFv, sFv, diabody, triabody, etc.) as long as it has the ability to specifically bind to CD3 expressed in target T cells that induce activation and to stimulate the surface molecules of these T cells to transmit a signal into the T cells.

For example, the molecule used in the activation culture of CAR-NK cells, preferably pluripotent stem cell (particularly iPS cell)-derived CAR-NK cells, is not particularly limited as long as it is a molecule that interacts with a surface molecule of CAR-NK cells and promotes the activation thereof. For example, it may be a physiological ligand (or receptor) for the above-mentioned NK cell surface molecule, or a non-physiological ligand (or receptor) having agonistic activity. It may also be a cytokine. A preferred example of the non-physiological ligand is an antibody against CD3. The antibody may be a complete antibody or a fragment thereof (e.g., Fab, F(ab')₂, Fab', scFv, Fv, reduced antibody (rIgG), dsFv, sFv, diabody, triabody, etc.). Examples of cytokines include IFN-α/β, IFN-γ, IL-2, IL-4, IL-7, IL-12, IL-15, IL-18, and IL-21.

### (Expansion step/expansion culture)

In this step, the cells activated as described above are proliferated. The method of expansion culture is not particularly limited as long as the desired effect on the activated cells is obtained, and a person skilled in the art can appropriately adjust the method while monitoring the number of cells, and the like. When the purpose is to increase the number of cells, the cells can be cultured in a medium containing the above-mentioned additives and the like for, for example, 2 days or more, preferably 3 days or more, more preferably 4 days or more, further preferably 5 days or more, and further more preferably 6 days or more. The cells can be cultured while monitoring the number of cells and the like and appropriately adjusting for, for example, 7 days or more, 9 days or more, or 11 days or more. In addition, the number of cells can be exponentially increased by continuing the culture by alternately repeating activation culture and expansion culture. The upper limit of the culture period is not particularly limited, and is, for example, 28 days or less, preferably 21 days or less. For example, in the case of activated CAR-T cells, preferably activated pluripotent stem cell (particularly iPS cell)-derived CAR-T cells, the cells can be expansion cultured in a medium containing cytokines such as IL-7, IL-15, and IL-2 for, for example, 2 days or more, preferably 3 days or more. When the target cells are primary T cells (particularly CAR-T cells) or NK cells (preferably pluripotent stem cell (particularly iPS cell-derived)-derived NK cells (particularly CAR-NK cells)) and the purpose is to increase the number of cells, the cells can be expansion cultured in a medium containing cytokines such as IL-7, IL-15, IL-21, and IL-2 for, for example, 2 days or more, preferably 4 days or more, more preferably 6 days or more, further preferably 8 days or more, further more preferably 10 days or more, and even further more preferably 11 days or more.

### (2) A method for producing a cell population (hereinafter also referred to as the "production method of the present invention") and a medicament containing the cell population (hereinafter to be also referred to as the "medicament of the present invention")

The production method of the present invention is a method for producing a cell population in which the number of desired cells is expanded, and the method includes a step of culturing a cell population containing the desired cells in a serum-free medium or low-serum medium containing (i) imidazole dipeptide or a salt thereof, (ii) taurine or a precursor thereof or a salt thereof, and (iii) lysophosphatidic acid or a derivative thereof, and further containing (iv) vitamin E and/or (v) boric acid or a salt thereof when desired.

Therefore, the production method may be a method including a step of culturing a cell population containing the desired cells in a serum-free medium or low-serum medium containing (ia) imidazole dipeptide or a salt thereof, or vitamin E, (ii) taurine or a precursor thereof, or a salt thereof, and (iii) lysophosphatidic acid or a derivative thereof.

The "serum-free medium or low-serum medium containing (i) imidazole dipeptide or a salt thereof, (ii) taurine or a precursor thereof or a salt thereof, and (iii) lysophosphatidic acid or a derivative thereof, and further containing (iv) vitamin E and/or (v) boric acid or a salt thereof when desired" and the "serum-free medium or low-serum medium containing (ia) imidazole dipeptide or a salt thereof, or vitamin E, (ii) taurine or a precursor thereof, or a salt thereof, and (iii) lysophosphatidic acid or a derivative thereof" have the same meaning as described in the culture method of the present invention and the proliferation method of the present invention in (1) above.

The desired cell is a cell the proliferation of which is aimed, and includes those exemplified in the above-mentioned section "1-1. Cell" in (1). It is preferably an immunocompetent cell, more preferably T cell or NK cell, particularly preferably T cell into which a CAR gene has been introduced, i.e., CAR-T cell, and NK cell into which a CAR gene has been introduced, i.e., CAR-NK cell. The CAR-T cells and CAR-NK cells are preferably derived from pluripotent stem cells, preferably iPS cells, and CAR-T cells derived from pluripotent stem cells (particularly iPS cells) are even more preferred.

The origin of the "cell population containing the desired cells" is not particularly limited as long as the desired cells are contained, and the cell population may be naturally derived or artificially prepared. For example, when immunocompetent cells such as T cells are desired, the cell population may be exemplified by cell populations containing peripheral blood mononuclear cells (PBMCs), blood cells, hematopoietic stem cells, cord blood mononuclear cells, and the like, collected, isolated, purified, or induced from body fluids such as blood (peripheral blood, umbilical cord blood, etc.) and bone marrow fluid. These cells may be collected from a living body or obtained through ex vivo culture and, for example, a cell population obtained by the production method of the present invention, either as is or frozen, can be mentioned. In addition, the cell population is not limited to a cell population of single cells, and may be a slice of a tissue mass obtained by biopsy, with preference given to a cell population of single cells. In the case of a humoral cell population, it is not limited to a cell population derived from a single tissue, and may be a mixed system of cell populations derived from different tissues. The "cell population containing the desired cells" is preferably a cell population containing immunocompetent cells, more preferably a cell population containing T cells or NK cells, and particularly preferably a cell population containing T cells into which a CAR gene has been introduced, i.e., CAR-T cells, or a cell population containing NK cells into which a CAR gene has been introduced, i.e., CAR-NK cells. A cell population containing CAR-T cells derived from pluripotent stem cells, preferably iPS cells, or a cell population containing CAR-NK cells derived from pluripotent stem cells, preferably iPS cells is even more preferred.

"The number of desired cells is expanded" refers to an increase in the number of desired cells in a cell population compared to a control, such as the number of cells before expansion or obtained without practicing the present invention, and means an increase of at least 10%, 20%, 30%, 40%, 50%, 60%, 70%, 80%, 90%, 100%, 120%, 150%, 200%, 300%, 400%, 500%, 600%, 700%, 800%, 900%, or 1000% compared to the control. In a specific embodiment of the present invention, the number of desired cells in the cell population produced by the present invention is expanded to achieve an increase by at least 10%, 20%, 30%, 40%, 50%, 60%, 70%, 80%, 90%, 100%, 120%, 150%, 200%, 300%, 400%, 500%, 600%, 700%, 800%, 900%, or 1000%, compared to the number of cells in the control.

When the desired cells are CAR-T cells or CAR-NK cells, the cells increase their number, i.e., are expanded, through activation culture and expansion culture (and recovery culture, where necessary), as described above. After expansion culture, the cells are washed and collected. When cultured or proliferated using the culture method and proliferation method of the present invention, since the medium used is a serum-free medium or a low-serum medium, the washing operation is simpler and the time required for washing can be shortened compared to when cultured or proliferated using a serum-containing medium (serum concentration: 5 to 20%) used generally.

For example, when cells are washed and collected using (i) pipetting and centrifugation or (ii) a closed-system automated cell processing device, continuous automated processing using techniques such as (i) pipetting and centrifugation or (ii) closed-system centrifugation/spinning membrane filtration/hollow fiber membrane filtration needs to be repeated multiple times until the BSA contained in the serum reaches an amount acceptable for administration to human. When cultured or proliferated using the culture method and proliferation method of the present invention, multiple repetitions are not necessary (the amount of BSA derived from serum brought into the culture system is originally small). Alternatively, the desired cell population can be collected by fewer repetitions compared to when a serum-containing medium (serum concentration: 5 to 20%) is used.

In one preferred embodiment, a step of culturing the cells (after expansion culture) in the presence of a stimulating substance is performed before the collection operation. This step allows efficient expansion culture, and also has the advantage of increasing the survival rate of the cells. When desired, the CAR-T cell population or CAR-NK cell population after expansion culture may be subjected to a washing operation and a cell separation step using beads separation or the like. This embodiment makes it possible to increase the purity of a CAR-T cell population or CAR-NK cell population with higher effects. Also in this embodiment, when cultured or expanded using the culture method and proliferation method of the present invention, the washing operation can be simplified and/or the time required for washing can be shortened.

In this way, the number of desired cells is expanded in the cell population obtained by the production method of the present invention.

The "cell population in which the number of desired cells is expanded" produced by the production method of the present invention can be used to produce a medicament as appropriate depending on the function of the cells. When the cells are CAR-T cells or CAR-NK cells, the cell population containing CAR-T cells or CAR-NK cells produced by the culture method and proliferation method of the present invention can be used to treat cancer, particularly cancer that expresses the target antigen of the CAR-expressing immune cells. The cancer may be a solid tumor or a blood tumor. Specific examples of cancer include, but are not limited to, various B-cell lymphomas (follicular malignant lymphoma, diffuse large B-cell malignant lymphoma, mantle cell lymphoma, MALT lymphoma, intravascular B-cell lymphoma, CD20-positive Hodgkin's lymphoma, etc.), myeloproliferative neoplasms, myelodysplastic/myeloproliferative neoplasms (CMML, JMML, CML, MDS/MPN-UC), myelodysplastic syndromes, acute myeloid leukemia, neuroblastoma, brain tumors, Ewing's sarcoma, osteosarcoma, retinoblastoma, small cell lung cancer, non-small cell lung cancer, melanoma, bone and soft tissue sarcoma, kidney cancer, pancreatic cancer, malignant mesothelioma, prostate cancer, breast cancer, uterine cancer, cervical cancer, ovarian cancer, and colorectal cancer. In a preferred embodiment, the cancer is a solid tumor. Examples of solid tumors include neuroblastoma, brain tumor, Ewing's sarcoma, osteosarcoma, retinoblastoma, small cell lung cancer, non-small cell lung cancer, melanoma, ovarian cancer, rhabdomyosarcoma, bone and soft tissue sarcoma, kidney cancer, pancreatic cancer, malignant mesothelioma, prostate cancer, breast cancer, uterine cancer, cervical cancer, ovarian cancer, and colorectal cancer.

The medicament of the present invention is administered in a therapeutically effective amount determined appropriately according to the age, weight, body surface area, symptoms, and the like of the subject. The subject in this disclosure is a mammal, particularly, generally a human, and preferably a cancer patient. The medicament of the present invention can be administered at, for example, 1x10⁴ cells to 1x10¹⁰ cells per administration. The administration route is not particularly limited, and it can be administered intratumorally, peritumorally, intraventricularly, intravenously, intraarterially, intraportally, intradermally, subcutaneously, intramuscularly, or intraperitoneally. The cell population of the present disclosure may be administered systemically or locally. Local administration includes direct injection into the target tissue, internal organ, or organ. The administration schedule is appropriately determined according to the age, weight, body surface area, symptoms, and the like of the subject, and may be a single administration or continuous or regular multiple administrations. The medicament of the present invention may be used for autotransplantation or allotransplantation. It may also be used in combination with other medicaments.

In particular, the medicament of the present invention may contain, in addition to the cell population to be administered to the subject, components such as dimethyl sulfoxide (DMSO) or serum albumin for the purpose of protecting the cells, antibiotics for the purpose of preventing bacterial contamination, and various components (vitamins, cytokines, growth factors, steroids, etc.) for the purpose of activating, proliferating, or inducing differentiation of cells. The composition can be prepared by a conventional method (for example, a method described in the Japanese Pharmacopoeia, etc.).

### (3) Medium additive (hereinafter to be also referred to as "medium additive of the present invention") and medium composition containing the medium additive (hereinafter to be also referred to as "medium composition of the present invention")

The medium additive of the present invention contains (i) imidazole dipeptide or a salt thereof, (ii) taurine or a precursor thereof, or a salt thereof, and (iii) lysophosphatidic acid or a derivative thereof, and further contains (iv) vitamin E and/or (v) boric acid or a salt thereof when desired.

Therefore, medium additive of the present invention may contain (ia) imidazole dipeptide or a salt thereof, or vitamin E, (ii) taurine or a precursor thereof, or a salt thereof, and (iii) lysophosphatidic acid or a derivative thereof.

The medium additive of the present invention is used for addition to a serum-free medium or a low-serum medium. The above-mentioned components (i) to (iii), and components (iv) and/or (v) used when desired, are the same as those described in the culture method of the present invention and the proliferation method of the present invention in the above-mentioned (1). The above-mentioned components (ia) to (iii) are the same as those described in the culture method of the present invention and the proliferation method of the present invention in the above-mentioned (1). The amount of each component to be added to the medium additive is not particularly limited as long as the desired effect is obtained. It may be generally 0.01 to 100% by weight, preferably 0.1 to 100% by weight, more preferably 1 to 100% by weight, further preferably 5 to 100% by weight, particularly preferably 10 to 100% by weight.

In the medium additive of the present invention, vitamin E may be used in addition to or instead of (i) imidazole dipeptide or a salt thereof, when desired, and when vitamin E is used, it is not necessary to further contain component (iv).

The medium composition of the present invention is a composition containing medium components obtained by adding the medium additive of the present invention to a serum-free medium or a low-serum medium. As used herein, the "serum-free medium or low-serum medium" has the same meaning as the "serum-free medium or low-serum medium" described in the above-mentioned (1) in the culture method of the present invention and the proliferation method of the present invention, except that components (i) to (iii) and, when desired, components (iv) and/or (v) have been removed. The medium additive of the present invention is added to a "serum-free medium or low-serum medium" such that the concentrations of components (i) to (iii), and when desired, components (iv) and/or (v) in the medium composition are as follows:
(i) imidazole dipeptides: generally 1 to 50 mM, preferably 3 to 40 mM, more preferably 5 to 30 mM, particularly preferably 10 to 25 mM.
(ii) taurines: generally 0.1 to 20 mM, preferably 0.5 to 15 mM, more preferably 1 to 10 mM, particularly preferably 2 to 5 mM.
(iii) lysophosphatidic acids: generally 0.1 to 50 mg/L, preferably 0.5 to 25 mg/L, more preferably 1 to 15 mg/L, particularly preferably 2 to 10 mg/L.
(iv) vitamin E: generally 10 to 1000 µM, preferably 20 to 500 µM, more preferably 40 to 300 µM, particularly preferably 100 to 250 µM.
(v) boric acid or a salt thereof: generally 0.1 to 0.9 mM, preferably 0.2 to 0.8 mM, more preferably 0.2 to 0.7 mM, particularly preferably 0.3 to 0.6 mM.

The medium composition of the present invention may be a composition containing medium components obtained by adding the medium additive of the present invention to a serum-free medium or a low-serum medium. As used herein, the "serum-free medium or low-serum medium" has the same meaning as the "serum-free medium or low-serum medium" described in the above-mentioned (1) in the culture method of the present invention and the proliferation method of the present invention, except that components (ia) to (iii) have been removed. The medium additive of the present invention is added to a "serum-free medium or low-serum medium" such that the concentrations of components (ia) to (iii) in the medium composition are as follows:
(ia) imidazole dipeptides: generally 1 to 50 mM, preferably 3 to 40 mM, more preferably 5 to 30 mM, particularly preferably 10 to 25 mM,
   vitamin E: generally 10 to 1000 µM, preferably 20 to 500 µM, more preferably 40 to 300 µM, particularly preferably 100 to 250 µM.
(ii) taurines: generally 0.1 to 20 mM, preferably 0.5 to 15 mM, more preferably 1 to 10 mM, particularly preferably 2 to 5 mM.
(iii) lysophosphatidic acids: generally 0.1 to 50 mg/L, preferably 0.5 to 25 mg/L, more preferably 1 to 15 mg/L, particularly preferably 2 to 10 mg/L.

When used in the present specification and claims, unless otherwise required by context, singular terms include plural terms and plural terms include singular terms. Thus, unless otherwise specified, singular articles (e.g., "a", "an", "the", etc. in the case of English) should be understood to also include the concept of plural forms thereof.

The present invention is described in detail in the following using Examples, but the present invention is not limited in any way. Unless otherwise specified, the reagents and materials to be used are commercially available or can be prepared according to known literatures and the like. Furthermore, those skilled in the art understand that any substance having a similar effect or action can be used alternatively.

### [Example]

### (List of Abbreviations)

BMP-4: bone morphogenetic protein-4
bFGF: basic fibroblast growth factor
VEGF: vascular endothelial growth factor
SCF: stem cell factor
TPO: thrombopoietin
FLT3L: Fms-related tyrosine kinase 3 ligand
Fc-DLL4: Recombinant Human DLL4 Fc Chimera Protein
DLL4: Delta-like protein 4
IL-7: Interleukin-7
IL-2: Interleukin-2
IL-15: Interleukin-15
IL-18: Interleukin-18
IL-21: Interleukin-21
SDF1α: Stromal cell-derived factor 1α
αMEM: alpha Modified Eagle Minimum Essential medium
IMDM: Iscove's Modified Dulbecco's medium
TCR: T-cell receptor
FBS: Fetal Bovine Serum
PBS: Phosphate-Buffered Saline
AA2P: L-Ascorbic acid 2-phosphate

### Example 1

### 1. Preparation of iPS cell-derived CAR-T cell

The TCR gene was introduced into the iPS cell line QHJI01S04 provided by the Center for iPS Cell Research and Application (CiRA), Kyoto University, using a lentiviral vector. The iPS cells into which the TCR gene was introduced were differentiated into hematopoietic progenitor cells with reference to the known method described in Nature Communication 2021; 12: 430. Specifically, the iPS cells were cultured for 4 days in the presence of CHIR99021, SB431542, BMP-4, bFGF, VEGF to induce differentiation into mesoderm. Furthermore, the hematopoietic cytokines SCF, TPO, and FLT3L were used to differentiate into hematopoietic progenitor cells. The differentiation of the obtained hematopoietic progenitor cells into cytotoxic T lymphocytes (CTLs) was performed according to a known patent method (WO2017/221975) and literature information (Nature Communication 2021; 12: 430). Specifically, from the obtained hematopoietic progenitor cells, CD34-positive cells were purified using magnetic beads (Myltenyi Biotec), and cultured for 3 weeks in α-MEM medium containing SCF, TPO, FLT3L, IL-7, SDF1α, and SB203580 on a plate on which Fc-DLL4 and RetroNectin (Recombinant Human Fibronectin Fragment, TakaraBio) were immobilized. A CAR gene that recognizes a specific antigen was introduced into the obtained TCR-positive CTL by using a retroviral vector. In the following, the cells were cryopreserved in liquid nitrogen and used as iPS cell-derived CAR-T cells.

### 2. Recovery culture of iPS cell-derived CAR-T cell

The iPS cell-derived CAR-T cells were suspended at 500,000 cells/mL in IMDM medium containing 15% FBS to which the additives shown in the "recovery culture medium" column in Table 1 were added, seeded on G-Rex (registered trademark) 6M (Wilson Wolf), and cultured for 3 days under 5% CO₂/37°C.

**[Table 1]**

| additive to medium | manufacturer | final concentration | recovery culture medium | activation culture medium | expansion culture medium 1 | expansion culture medium 2 |
|---|---|---|---|---|---|---|
| L-Glutamine (200 mM) | Thermo Fisher | 2 mM | + | + | + | + |
| Streptomycin sulfate | MEIJI | 100 µg/mL | + | + | + | + |
| ITS (100x) (Insulin-Transferrin-Selenium supplements) | Gibco | 1x | + | + | + | + |
| L-Ascorbic acid 2-phosphate sesquinagnesium salt hydrate | Sigma-Aldrich | 50 µg/mL | + | + | + | + |
| IL-7 | Peprotech | 10 ng/mL | + | + | + | + |
| IL-15 | Peprotech | 10 ng/mL | + | + | + | + |
| IL-2 | Peprotech | 100 ng/mL | + | + | + | + |
| IL-18 | MBL | 50 ng/mL | | + | | |
| IL-21 | Peprotech | 20 ng/mL | | + | + | + |
| Caspase Inhibitor Z-VAD-FMK | R&D | 10 µM | | + | | |
| Human CD30 antibody | R&D | 300 ng/mL | | + | + | + |
| Albuminar 25% I.V. Injection 12.5 g/50 mL (Human Serum Albumin, HSA) | CSL Behring | 2 g/L | | | | + |
| Boric acid | Sigma-Aldrich | 0.3 mM | | | | + |
| ITS-X (100x) (Insulin-Transferrin-Selenium-Ethanolamine supplements) | Gibco | 1x | | | | + |
| Taurine | Sigma-Aldrich | 3 mM | | | | + |
| LPC (soybean) (L-alpha-Lysophosphatidylcholine) | Sigma-Aldrich | 5 mg/L | | | | + |

| | | | | | | |
|---|---|---|---|---|---|---|
| Additives added to each group are indicated with +. | | | | | | |

### 3. Reagent, antibody

The anti-CD3 agonist antibody used was Anti-CD3 mAb GMP grade, Anti-CD3 monoclonal antibody (Clone: OKT3) purchased from Takara Bio Inc.

### 4. Immobilization of anti-CD3 agonist antibody on culture plate

Anti-CD3 agonist antibody (OKT3, final concentration 3 µg/mL) dissolved in PBS at the required concentration was added to a T225 flask and left standing overnight at 4°C, After washing with PBS, the cells were subjected to the test.

### 5. Activation culture of iPS cell-derived CAR-T cell

The iPS cell-derived CAR-T cells after recovery culture were suspended at 133,333 cells/mL in IMDM medium containing 15% FBS to which the additives shown in the "activation culture medium" column in Table 1 were added, seeded into a T225 flask to which an anti-CD3 agonist antibody (OKT3) was immobilized, and cultured for 3 days under 5% CO₂/37°C.

### 6. Expansion culture of iPS cell-derived CAR-T cells in three types of serum-free media

A total of three types of expansion culture media were prepared: a medium containing the components listed in Table 2 (hereinafter to be referred to as BM220720), and further, the additives listed in the "expansion culture medium 2" column in Table 1, and said medium added with 20 mM carnosine (Sigma-Aldrich) or 100 µM α-tocopherol (Sigma-Aldrich). As an experimental control, a medium was prepared by adding the additives listed in the "expansion culture medium 1" column in Table 1 to IMDM medium containing 15% FBS. After 3 days of activation culture, the cells were collected from the T225 flask and suspended in each of the four types of expansion culture media at 40,000 cells/mL, and cultured under 5% CO₂/37°C using G-Rex (registered trademark) 6M (Wilson Wolf). Then, on the second day of culture, some of the cells were recovered from G-Rex (registered trademark) 6M and the cells were counted, and the medium was replaced with the respective medium. On the third day of culture, some of the cells were recovered from G-Rex (registered trademark) 6M and the cells were counted. On the fourth day of culture, some of the cells were recovered from G-Rex (registered trademark) 6M and the cells were counted.

**[Table 2-1]**

| **component name** | **concentration (mg/L)** |
|---|---|
| Glycine | 30 |
| L-Alanine | 25 |
| L-Arginine hydrochloride | 84 |
| L-Asparagine (freebase) | 25 |
| L-Aspartic acid | 30 |
| L-Cystine 2HCl | 91.4 |
| L-Glutamic Acid | 75 |
| L-Histidine hydrochloride-H₂O | 42 |
| L-Isoleucine | 105 |
| L-Leucine | 105 |
| L-Lysine hydrochloride | 146 |
| L-Methionine | 30 |
| L-Phenylalanine | 66 |
| L-Proline | 40 |
| L-Serine | 42 |
| L-Threonine | 95 |
| L-Tryptophan | 16 |
| L-Tyrosine 2Na 2H₂O | 121 |
| L-Valine | 94 |
| L-Ascorbic Acid 2-Phosphate Sesquimagnesium Salt | 2.5 |
| Biotin | 0.013 |
| Choline chloride | 8.98 |
| D-Calcium pantothenate | 4 |
| Folic Acid | 4 |
| Niacinamide | 4 |
| Pyridoxine hydrochloride | 2.013 |
| Riboflavin | 0.4 |

**[Table 2-2]**

| | |
|---|---|
| Thiamine hydrochloride | 4 |
| Vitamin B12 | 0.68 |
| i-Inositol | 12.6 |
| Calcium Chloride (CaCl₂) (anhyd.) | 165 |
| Cupric sulfate (CuSO₄ 5H₂O) | 0.0013 |
| Ferric Nitrate (Fe(NO₃)₃9H₂O) | 0.05 |
| Ferric sulfate (FeSO₄ 7H₂O) | 0.417 |
| Magnesium Sulfate (MgSO₄) (anhyd.) | 97.67 |
| Potassium Chloride (KCI) | 330 |
| Sodium Bicarbonate (NaHCO₃) | 3024 |
| Sodium Chloride (NaCl) | 4350 |
| Sodium Phosphate monobasic (NaH₂PO₄ H₂O) | 125 |
| Zinc sulfate (ZnSO₄ 7H₂O) | 0.864 |
| Sodium Selenite | 0.005 |
| D-Glucose (Dextrose) | 4500 |
| HEPES | 5958 |
| Ethanolamine | 1.9 |
| Hypoxanthine Na | 2.39 |
| Linoleic Acid | 0.042 |
| Lipoic Acid | 0.105 |
| Putrescine 2HCl | 0.081 |
| Sodium Pyruvate | 110 |
| Thymidine | 0.365 |
| L-Glutathione reduced | 1 |
| Ammonium Metavanadate | 0.0003 |
| Manganous Chloride tetrahydrate (MnCl₂ 4H₂O) | 0.00005 |

### Experimental Example 1-1

### Proliferation test to verify the effect of adding carnosine or α-tocopherol to serum-free medium

In the expansion culture of item 6 in Example 1, the cell number and survival rate of iPS cell-derived CAR-T cells were measured on days 2, 3, and 4 of expansion culture. The cell proliferation fold from day 0 of expansion culture is shown in Table 3. The proliferation of iPS cell-derived CAR-T cells was promoted by adding 20 mM carnosine or 100 µM α-tocopherol to a serum-free medium (Table 3). The addition of 20 mM carnosine to serum-free medium resulted in a higher proliferation fold than in a medium containing 15% FBS. The cell survival rate of cells from day 0 of expansion culture is shown in Table 4. The survival rate of iPS cell-derived CAR-T cells was improved by adding 20 mM carnosine or 100 µM α-tocopherol to the serum-free medium (Table 4).

**[Table 3]**

| conditions of expansion culture medium | proliferation fold from day 0 of expansion culture | | | |
|---|---|---|---|---|
| | day 0 | day 2 | day 3 | day 4 |
| IMDM + 15% FBS + Gln + AA2P + IL-2/IL-7/IL-15/IL21 + CD30 Ab + ITS | 1.00 | 7.25 | 15.3 | 26.1 |
| BM220720 + Gln + AA2P + IL-2/IL-7/IL-15/IL-21 + CD30 Ab + ITS + HSA + ITS-X + Boric Acid + Taurine + 5 mg/L LPC | 1.00 | 8.07 | 14.5 | 21.2 |
| BM220720 + Gln + AA2P + IL-2/IL-7/IL-15/IL-21 + CD30 Ab + ITS + HSA + ITS-X + Boric Acid + Taurine + 5 mg/L LPC + 20 mM carnosine | 1.00 | 8.56 | 18.6 | 32.3 |
| BM220720 + Gln + AA2P + IL-2/IL-7/IL-15/IL-21 + CD30 Ab + ITS + HSA + ITS-X + Boric Acid + Taurine + 5 mg/L LPC + 100 µM α-Tocopherol | 1.00 | 8.54 | 16.9 | 26.6 |

**[Table 4]**

| conditions of expansion culture medium | cell survival rate (%) | | | |
|---|---|---|---|---|
| | day 0 | day 2 | day 3 | day 4 |
| IMDM + 15% FBS + Gln + AA2P + IL-2/IL-7/IL-15/IL-21 + CD30 Ab + ITS | 94.5 | 91.1 | 91.9 | 92.5 |
| BM220720 + Gln + AA2P + IL-2/IL-7/IL-15/IL-21 + CD30 Ab + ITS + HSA + ITS-X + Boric Acid + Taurine + 5 mg/L LPC | 94.5 | 95.1 | 86.2 | 84.6 |
| BM220720 + Gln + AA2P + IL-2/IL-7/IL-15/IL-21 + CD30 Ab + ITS + HSA + ITS-X + Boric Acid + Taurine + 5 mg/L LPC + 20 mM carnosine | 94.5 | 94.4 | 88.5 | 88.4 |
| BM220720 + Gln + AA2P + IL-2/IL-7/IL-15/IL-21 + CD30 Ab + ITS + HSA + ITS-X + Boric Acid + Taurine + 5 mg/L LPC + 100 µM α-Tocopherol | 94.5 | 95.5 | 89.3 | 89.2 |

### Experimental Example 1-2

### Test to confirm proliferation ability of cryopreserved cells in serum-containing medium

In the expansion culture of item 6 of Example 1, cells were collected on the third day of expansion culture of iPS cell-derived CAR-T cells, and the cells were counted. A suspension containing a specified number of cells was centrifuged at 300xg for 5 minutes, and the supernatant was removed. The cell aggregate was suspended at 8,000,000 cells/mL in MEM medium to which Albumin 25% I.V. Injection 12.5 g/50 mL (product name) was added to a final albumin concentration of 5%, an equal amount of CryoStor (registered trademark) CS10 (STEMCELL Technologies) was added to this suspension, and the mixture was cryopreserved. After thawing the cryopreserved cells, the cells were suspended in IMDM medium containing 15% FBS, 2 mM glutamine, ITS (x1), and 50 µg/mL ascorbic acid 2-phosphate sesquimagnesium salt hydrate at 125,000 cells/mL, and cultured under 5% CO₂/37°C by using a G-Rex 24-well plate. The number of viable cells and cell survival rate were measured on days 0, 3, and 7 of culture. The measurement results are shown in Table 5. Cells cultured in expansion culture medium containing 20 mM carnosine or 100 µM α-tocopherol and then cryopreserved showed improved survival rates from day 0 to day 7 after thawing in the proliferation ability confirmation test, and the proliferation fold was also improved (Table 5). However, the proliferation fold was lower than that of cells expanded in a medium containing 15% FBS.

**[Table 5]**

| conditions of expansion culture medium before cell freezing | proliferation fold from day 0 after thawing | | | cell survival rate (%) | | |
|---|---|---|---|---|---|---|
| | day 0 | day 3 | day 7 | day 0 | day 3 | day 7 |
| IMDM + 15% FBS + Gln + AA2P + IL-2/IL-7/IL-15/IL-21 + CD30 Ab + ITS | 1.00 | 7.16 | 64.8 | 90.9 | 94.8 | 99.1 |
| BM220720 + Gln + AA2P + IL-2/IL-7/IL-15/IL-21 + CD30 Ab + ITS + ITS-X + HSA + Boric Acid + Taurine + 5 mg/L LPC | 1.00 | 4.76 | 34.8 | 88.0 | 94.7 | 98.8 |
| BM220720 + Gln + AA2P + IL-2/IL-7/IL-15/IL-21 + CD30 Ab + ITS + HSA + ITS-X + Boric Acid + Taurine + 5 mg/L LPC + 20 mM carnosine | 1.00 | 5.74 | 41.0 | 93.0 | 97.7 | 99.8 |
| BM220720 + Gln + AA2P + IL-2/IL-7/IL-15/IL-21 + CD30 Ab + ITS + HSA + ITS-X + Boric Acid + Taurine + 5 mg/L LPC + 100 µM α-Tocopherol | 1.00 | 5.30 | 39.9 | 91.0 | 96.7 | 99.2 |

### Example 2

### 1. Recovery culture of iPS cell-derived CAR-T cell

The iPS cell-derived CAR-T cells were suspended at 500,000 cells/mL in IMDM medium containing 15% FBS to which the additives shown in the "recovery culture medium" column in Table 6 were added, seeded on G-Rex (registered trademark) 6M (Wilson Wolf), and cultured for 3 days under 5% CO₂/37°C.

**[Table 6]**

| additive to medium | manufacturer | final concentration | recovery culture medium | activation culture medium | expansion culture medium 1 | expansion culture medium 2 |
|---|---|---|---|---|---|---|
| L-Glutamine (200 mM) | Thermo Fisher | 2 mM | + | + | + | + |
| Streptomycin sulfate | MEIJI | 100 µg/mL | + | + | + | + |
| ITS (100x) (Insulin-Transferrin-Selenium supplements) | Gibco | 1x | + | + | + | + |
| L-Ascorbic acid 2-phosphate sesquimagnesium salt hydrate | Sigma-Aldrich | 50 µg/mL | + | + | + | + |
| IL-7 | Peprotech | 10 ng/mL | + | + | + | + |
| IL-15 | Peprotech | 10 ng/mL | + | + | + | + |
| IL-2 | Peprotech | 100 ng/mL | + | + | + | + |
| IL-18 | MBL | 50 ng/mL | | + | | |
| IL-21 | Peprotech | 20 ng/mL | | + | + | + |
| Caspase Inhibitor Z-VAD-FMK | R&D | 10 µM | | + | | |
| Human CD30 antibody | R&D | 300 ng/mL | | + | + | + |
| Albuminar 25% I.V. Injection 12.5 g/50 mL (Human Serum Albumin, HSA) | CSL Behring | 2 g/L | | | | + |
| Boric acid | Sigma-Aldrich | 0.3 mM | | | | + |
| ITS-X (100x) (Insulin-Transferrin-Selenium-Ethanolamine supplements) | Gibco | 1x | | | | + |
| Taurine | Sigma-Aldrich | 3 mM | | | | + |
| LPC (soybean) (L-alpha-Lysophosphatidylcholine) | Sigma-Aldrich | 5 mg/L | | | | + |

| | | | | | | |
|---|---|---|---|---|---|---|
| Additives added to each group are indicated with +. | | | | | | |

### 2. Reagent, antibody

The anti-CD3 agonist antibody used was Anti-CD3 mAb GMP grade, Anti-CD3 monoclonal antibody (Clone: OKT3) purchased from Takara Bio Inc.

### 3. Immobilization of anti-CD3 agonist antibody on culture plate

Anti-CD3 agonist antibody (OKT3, final concentration 3 µg/mL) dissolved in PBS at the required concentration was added to a T225 flask and left standing overnight at 4°C, After washing with PBS, the cells were subjected to the test.

### 4. Activation culture of iPS cell-derived CAR-T cell

The iPS cell-derived CAR-T cells after recovery culture were suspended at 133,333 cells/mL in IMDM medium containing 15% FBS to which the additives shown in the "activation culture medium" column in Table 6 were added, seeded into a T225 flask to which an anti-CD3 agonist antibody (OKT3) was immobilized, and cultured for 3 days under 5% CO₂/37°C.

### 5. Expansion culture of iPS cell-derived CAR-T cells in two types of serum-free media

A total of two types of serum-free expansion culture media were prepared: BM220720 medium containing the components listed in the "expansion culture medium 2" column in Table 6, and further, 20 mM carnosine (Sigma-Aldrich), and a medium added with 20 mM carnosine and 200 µM α-tocopherol (Sigma-Aldrich). As an experimental control, a medium was prepared by adding the additives listed in the "expansion culture medium 1" column in Table 6 to IMDM medium containing 15% FBS. After 3 days of activation culture, the cells were collected from the T225 flask and suspended in each of the three types of expansion culture media at 40,000 cells/mL, and cultured under 5% CO₂/37°C using G-Rex (registered trademark) 100M (Wilson Wolf). Then, on the second day of culture, some of the cells were recovered from G-Rex (registered trademark) 100M and the cells were counted, and the medium was replaced with the respective medium. On the second day of culture, some of the cells were recovered from G-Rex (registered trademark) 100M and the cells were counted. On the third day of culture, some of the cells were recovered from G-Rex (registered trademark) 100M and the cells were counted.

### Experimental Example 2-1

### Proliferation test to verify the additive effect of carnosine and α-tocopherol in serum-free medium

In order to verify the additive effect of 20 mM carnosine and 200 µM α-tocopherol in serum-free expansion culture medium, in the expansion culture of item 5 in Example 2, the cell number and survival rate of iPS cell-derived CAR-T cells were measured on days 2 and 3 of culture. The cell proliferation fold from day 0 of expansion culture is shown in Table 7. The iPS cell-derived CAR-T cells showed a higher proliferation fold in a serum-free medium than in a medium containing 15% FBS under all conditions (Table 7). The cell survival rate from day 0 of expansion culture is shown in Table 8. The survival rate of the iPS cell-derived CAR-T cells in a serum-free medium was 90% or higher under all conditions (Table 8).

**[Table 7]**

| conditions of expansion culture medium | proliferation fold from day 0 | | |
|---|---|---|---|
| | day 0 | day 2 | day 3 |
| IMDM + 15% FBS + Gln + AA2P + IL-2/IL-7/IL-15/IL-21+ CD30 Ab + ITS | 1.00 | 7.35 | 16.5 |
| BM220720 + Gln + AA2P + IL-2/IL-7/IL-15/IL-21 + CD30 Ab + ITS + HSA + ITS-X + Boric Acid + Taurine + 5 mg/L LPC + 20 mM carnosine | 1.00 | 7.65 | 17.9 |
| BM220720 + Gln + AA2P + IL-2/IL-7/IL-15/IL-21 + CD30 Ab + ITS + HSA + ITS-X + Boric Acid + Taurine + 5 mg/L LPC + 20 mM carnosine + 200 µM α-Tocopherol | 1.00 | 7.51 | 17.4 |

**[Table 8]**

| conditions of expansion culture medium | cell survival rate (%) | | |
|---|---|---|---|
| | day 0 | day 2 | day 3 |
| IMDM + 15% FBS + Gln + AA2P + IL-2/IL-7/IL-15/IL-21+ CD30 Ab + ITS | 95.70 | 95.25 | 95.6 |
| BM220720 + Gln + AA2P + IL-2/IL-7/IL-15/IL-21 + CD30 Ab + ITS + HSA + ITS-X + Boric Acid + Taurine + 5 mg/L LPC + 20 mM carnosine | 95.70 | 92.15 | 90.8 |
| BM220720 + Gln + AA2P + IL-2/IL-7/IL-15/IL-21 + CD30 Ab + ITS + HSA + ITS-X + Boric Acid + Taurine + 5 mg/L LPC + 20 mM carnosine + 200 µM α-Tocopherol | 95.70 | 90.35 | 90.2 |

### Experimental Example 2-2

### Test to confirm proliferation ability of cryopreserved cells in serum-containing medium

In the expansion culture of item 5 of Example 2, cells were collected on the third day of expansion culture of iPS cell-derived CAR-T cells, and the cells were counted. A suspension containing a specified number of cells was centrifuged at 300xg for 5 minutes, and the supernatant was removed. The cell aggregate was suspended at 8,000,000 cells/mL in MEM medium to which Albumin 25% I.V. Injection 12.5 g/50 mL (product name) was added to a final albumin concentration of 5%, an equal amount of CS10 was added to this suspension, and the mixture was cryopreserved. After thawing the cryopreserved cells, the cells were suspended in IMDM medium containing 15% FBS, 2 mM glutamine, ITS (x1), and 50 µg/mL ascorbic acid 2-phosphate sesquimagnesium salt hydrate at 125,000 cells/mL, and cultured under 5% CO₂/37°C by using a G-Rex 24-well plate. The number of viable cells and cell survival rate were measured on days 0, 3, and 7 of culture. The measurement results are shown in Table 9. Cells cultured in an expansion culture medium added 20 mM carnosine and 200 µM α-tocopherol and then cryopreserved showed improved proliferation fold in the proliferation ability confirmation test, and the proliferation fold was higher than the cells cultured in an expansion culture medium containing 15% FBS and then cryopreserved. In this test, an additive effect of carnosine and α-tocopherol was observed in the expansion culture (Table 9).

**[Table 9]**

| conditions of expansion culture medium before cell freezing | proliferation fold from day 0 after thawing | | | cell survival rate (%) | | |
|---|---|---|---|---|---|---|
| | day 0 | day 3 | day 7 | day 0 | day 3 | day 7 |
| IMDM + 15% FBS + Gln + AA2P + IL-2/IL-7/IL-15/IL-21 + CD30 Ab + ITS | 1.00 | 6.72 | 50.3 | 85.6 | 92.3 | 99.2 |
| BM220720 + Gln + AA2P + IL-2/IL-7/IL-15/IL-21 + CD30 Ab + ITS + HSA + ITS-X + Boric Acid + Taurine + 5 mg/L LPC + 20 mM carnosine | 1.00 | 6.67 | 40.4 | 88.9 | 98.5 | 98.9 |
| BM220720 + Gln + AA2P + IL-2/IL-7/IL-15/IL-21 + CD30 Ab + ITS + HSA + ITS-X + Boric Acid + Taurine + 5 mg/L LPC + 20 mM carnosine + 200 µM α-Tocopherol | 1.00 | 11.20 | 69.0 | 90.7 | 97.3 | 99.3 |

### Example 3

### 1. Activation culture of human-derived primary T cell

Human-derived primary T cells (Leukopak-SoloPak, Charles River Laboratories Cell Solutions) were suspended at 1,000,000 cells/mL in OpTmizer medium (Thermo Fisher) containing 2% CTS Immune Cell SR (Thermo Fisher) and the additives shown in the activation culture medium in Table 10, seeded in a PL240 bag (OriGen), and cultured for 2 days under 5% CO₂/37°C.

**[Table 10]**

| additive to medium | manufacturer | final concentration | activation culture medium | transduction culture medium | expansion culture medium 1 | expansion culture medium 2 |
|---|---|---|---|---|---|---|
| OpTmizer Expansion Basal Supplement | Thermo Fisher | | + | + | + | |
| TransACT | Miltenyi Biotec | 2/37 volume | + | | | |
| L-Glutamine (200 mM) | Thermo Fisher | 2 mM | + | + | + | + |
| Streptomycin sulfate | MEIJI | 100 µg/mL | + | + | + | + |
| ITS-X (100x) (Insulin-Transferrin-Selenium-Ethanolamine supplements) | Gibco | 1x | | | | + |
| L-Ascorbic acid 2-phosphate Sesquimagnesium salt hydrate | Sigma-Aldrich | 50 µg/mL | | | | + |
| IL-2 | Miltenyi Biotec | 40 IU/mL | + | + | + | + |
| Albuminar 25% I.V. Injection 12.5 g/50 mL (Human Serum Albumin, HSA) | CSL Behring | 2 g/L | | | | + |

| | | | | | | |
|---|---|---|---|---|---|---|
| Additives added to each group are indicated with +. | | | | | | |

### 2. Reagent

RetroNectin (trade name) was purchased from Takara Bio Inc.

### 3. Immobilization of RetroNectin (product name) and retrovirus on culture plate

RetroNectin (final concentration 20 µg/mL) dissolved in PBS at the necessary concentration was added to a PL120 bag and left standing at room temperature for 2 hr. After washing with PBS, a retrovirus solution was added and immobilized overnight at 4°C while shaking at 50 rpm. After washing with PBS containing 1.5% HSA, the plate was subjected to the test.

### 4. Transduction culture of human-derived primary T cell

The human-derived primary T cells after activation culture were suspended at 722,222 cells/mL in OpTmizer medium containing 2% CTS Immune Cell SR and the additives shown in the transduction culture medium in Table 10, seeded in a PL120 bag in which RetroNectin (trade name) and a retroviral vector containing a CAR gene that recognizes a specific antigen were immobilized, and cultured for one day under 5% CO₂/37°C.

### 5. Expansion culture of human-derived primary T cell-derived CAR-T cells in three types of serum-free media

A total of three types of expansion culture media were prepared: an expansion culture medium which was OpTmizer medium containing 2% CTS Immune Cell SR and the additives shown in expansion culture medium 1 in Table 10, an expansion culture medium which was BM220720 medium containing the additives shown in expansion culture medium 2 in Table 10, and said medium further added with 0.3 mM boric acid (Sigma-Aldrich) and 3 mM taurine (Sigma-Aldrich), 5 mg/L lysophosphatidylcholine (Sigma-Aldrich), 20 mM carnosine (Sigma-Aldrich), 200 µM α-tocopherol (Sigma-Aldrich). After 1 day of transduction culture, the cells were collected from the PL120 bag and suspended in each of the expansion culture media at 220,000 cells/mL, and expansion cultured under 5% CO₂/37°C using G-Rex (registered trademark) 6M (Wilson Wolf). Then, on the fourth day of expansion culture, some of the cells were recovered from G-Rex (registered trademark) 6M and the cells were counted, and 40 IU of IL-2 was added to each medium. On the sixth day of expansion culture, some of the cells were recovered from G-Rex (registered trademark) 6M and the cells were counted, and seeded at 220,000 cells/mL in fresh G-Rex (registered trade mark) 6M containing respective medium. On the tenth day of expansion culture, some of the cells were recovered from G-Rex (registered trademark) 6M and the cells were counted, and 40 IU of IL-2 was added to each medium. On the eleventh day, some of the cells were recovered from G-Rex (registered trademark) 6M and the cells were counted.

### Experimental Example 3

### Proliferation test to verify effect of adding boric acid, taurine, lysophosphatidylcholine, carnosine, and α-tocopherol to human-derived primary T cell-derived CAR-T cell

To verify the effect of adding boric acid, taurine, lysophosphatidylcholine, carnosine, and α-tocopherol to serum-free expansion culture medium on human-derived primary T cells, the cell number and survival rate of human-derived primary T cell-derived CAR-T cells were measured on days 4, 6, 10, and 11 in the expansion culture described in item 5 of Example 3. The cell proliferation fold from day 0 of expansion culture is shown in Table 11. The proliferation of human-derived primary T cell-derived CAR-T cells was promoted by adding 0.3 mM boric acid, 3 mM taurine, 5 mg/L lysophosphatidylcholine, 20 mM carnosine, and 200 µM α-tocopherol to a serum-free medium (Table 11). The cell survival rate from day 0 of expansion culture is shown in Table 12. The survival rate of human-derived primary T cell-derived CAR-T cells from day 10 of expansion culture onwards was improved by adding 0.3 mM boric acid, 3 mM taurine, 5 mg/L lysophosphatidylcholine, 20 mM carnosine, and 200 µM α-tocopherol to a serum-free medium (Table 12).

**[Table 11]**

| conditions of expansion culture medium | proliferation fold from day 0 | | | | |
|---|---|---|---|---|---|
| | day 0 | day 4 | day 6 | day 10 | day 11 |
| OpTmizer + 2.6% OpTmizer Expansion Suplement + 2% CTS Immune Cell SR + Gln + AA2P + IL-2 | 1.00 | 17.39 | 21.1 | 42.6 | 55.0 |
| BM220720 + Gln + AA2P + IL-2 + HSA + ITS-X | 1.00 | 16.71 | 18.1 | 56.3 | 70.2 |
| BM220720 + Gln + AA2P + IL-2 + HSA + ITS-X + Boric Acid + Taurine + 5 mg/L LPC + 20 mM carnosine + 200 µM α-Tocopherol | 1.00 | 16.71 | 17.5 | 84.7 | 112.3 |

**[Table 12]**

| conditions of expansion culture medium | cell survival rate (%) | | | | |
|---|---|---|---|---|---|
| | day 0 | day 4 | day 6 | day 10 | day 11 |
| OpTmizer + 2.6% OpTmizer Expansion Suplement + 2% CTS Immune Cell SR + Gln + AA2P + IL-2 | 96.9 | 92.4 | 89.8 | 82.2 | 83.2 |
| BM220720 + Gln + AA2P + IL-2 + HSA + ITS-X | 96.9 | 89.7 | 85.8 | 87.9 | 89.1 |
| BM220720 + Gln + AA2P + IL-2 + HSA + ITS-X + Boric Acid + Taurine + 5 mg/L LPC + 20 mM carnosine + 200 µM α-Tocopherol | 96.9 | 89.5 | 82.9 | 93.3 | 95.2 |

### Example 4

### 1. Preparation of iPS cell-derived NK cell

The iPS cell line FfI01s04 provided by the Center for iPS Cell Research and Application (CiRA), Kyoto University, was differentiated into NK cells according to a known method (e.g., the method described in WO2021/174004). The obtained cells were frozen and stored in liquid nitrogen and used as iPS cell-derived NK cells.

### 2. Recovery culture of iPS cell-derived NK cell

iPS cell-derived NK cells were suspended in a recovery culture medium, which was IMDM medium (Thermo Fisher Scientific) containing 15% FBS and the additives shown in Table 13, at 1.25×10⁵ cells/mL, seeded in a G-Rex (registered trademark) 6-well plate (Wilson Wolf), and cultured for 3 days in a 5% CO₂/37°C incubator (culture days 0 to 3).

**[Table 13]**

| additive to medium | manufacturer | final concentration |
|---|---|---|
| L-Glutamine-Penicillin-Streptomycin solution | Sigma Aldrich | 1% |
| ITS (100x) (Insulin-Transferrin-Selenium supplements) | Gibco | 1x |
| L-Ascorbic acid 2-phosphate sesquimagnesium salt hydrate | Sigma-Aldrich | 50 µg/mL |
| IL-2 | PeproTech | 10 ng/mL |
| IL-7 | PeproTech | 10 ng/mL |
| IL-15 | PeproTech | 10 ng/mL |

### 3. Activation culture of iPS cell-derived NK cell

After recovery culture, the iPS cell-derived NK cells were suspended in an activation culture medium, which was IMDM medium (Thermo Fisher Scientific) containing 15% FBS and the additives shown in Table 14, at 1.84×10⁵ cells/mL, seeded in a 6-well plate (TPP), and cultured for 3 days in a 5% CO₂/37°C incubator (culture days 3-6).

**[Table 14]**

| additive to medium | manufacturer | final concentration |
|---|---|---|
| L-Glutamine-Penicillin-Streptomycin solution | Sigma Aldrich | 1% |
| ITS (100x) (Insulin-Transferrin-Selenium supplements) | Gibco | 1x |
| L-Ascorbic acid 2-phosphate sesquimagnesium salt hydrate | Sigma-Aldrich | 50 µg/mL |
| IL-2 | PeproTech | 10 ng/mL |
| IL-7 | PeproTech | 10 ng/mL |
| IL-12 | Proteintech Group | 50 ng/mL |
| IL-15 | Peprotec | 10 ng/mL |
| IL-18 | MBL | 50 ng/mL |
| IL-21 | PeproTech | 20 ng/mL |
| Human agonistic Antibody | R&D Systems | 300 ng/mL |
| Pan Caspase Inhibitor Z-VAD-FMK | R&D Systems | 10 µM |

### 4. Expansion culture of iPS cell-derived NK cell in three types of serum-free media and one type of serum-containing medium

After 3 days, a total of four types of expansion culture media were produced: a basal medium (first type of serum-free medium) which was BM220720 medium of Table 2 added with human serum albumin (Albuminar 25% I.V. Injection 12.5 g/50 mL (product name)) at a final concentration of 2 g/L, a basal medium (second type of serum-free medium) which was BM220720 medium added with human serum albumin (Albuminar 25% I.V. Injection 12.5 g/50 mL (product name)) at a final concentration of 2 g/L, boric acid (Sigma-Aldrich) at a final concentration of 0.3 mM, taurine (Sigma-Aldrich) at a final concentration of 3 mM, lysophosphatidylcholine (Sigma-Aldrich) at a final concentration of 5 mg/L, carnosine (Sigma-Aldrich) at a final concentration of 20 mM, and α-tocopherol (Sigma-Aldrich) at a final concentration of 200 µM, a basal medium (third type of serum-free medium) based on X-VIVO (trademark) 15 medium (Lonza), and a basal medium which was IMDM medium (Thermo Fisher Scientific) added with FBS at a final concentration of 15%, and the additives shown in Table 14 were added to the basal media to give four types of expansion culture media. The cells that had been activation cultured for three days were suspended in each of the four types of expansion culture media at 1.25×10⁵ cells/mL, reseeded into G-Rex (registered trademark) 24-well plates (Wilson Wolf), and cultured in a 5% CO₂/37°C incubator for two days (culture days 6-8).

After two days, the media were replaced with the four types of basal media supplemented with the additives shown in Table 15, and the cells were cultured for two days in a 5% CO₂/37°C incubator (culture days 8-10).

Thereafter, the cells were reseeded in the same medium every 2-3 days onto a G-Rex (registered trademark) 24-well plate (Wilson Wolf) at 1.25×10⁵ cells/mL, and culture was continued in a 5% CO₂/37°C incubator until culture day 17.

**[Table 15]**

| additive to medium | manufacturer | final concentration |
|---|---|---|
| L-Glutamine-Penicillin-Streptomycin solution | Sigma Aldrich | 1% |
| ITS (100x) (Insulin-Transferrin-Selenium supplements) | Gibco | 1x |
| L-Ascorbic acid 2-phosphate sesquimagnesium salt hydrate | Sigma-Aldrich | 50 µg/mL |
| IL-2 | PeproTech | 10 ng/mL |
| IL-7 | PeproTech | 10 ng/mL |
| IL-15 | PeproTech | 10 ng/mL |
| IL-21 | PeproTech | 20 ng/mL |

### Experimental Example 4

### Proliferation test to verify the effect of adding boric acid, taurine, lysophosphatidylcholine, carnosine, and α-tocopherol to iPS cell-derived NK cell

To verify the effect of adding boric acid, taurine, lysophosphatidylcholine, carnosine, and α-tocopherol to serum-free expansion culture medium on iPS cell-derived NK cells, the cell number and survival rate of iPS cell-derived NK cells were measured over time during the expansion culture in item 4 of Example 4. The proliferation fold and cell survival rate on day 11 of expansion culture are shown in Table 16. The proliferation of iPS cell-derived NK cells increased by 7 times or more by adding 0.3 mM boric acid (Sigma-Aldrich), 3 mM taurine (Sigma-Aldrich), 5 mg/L lysophosphatidylcholine (Sigma-Aldrich), 20 mM carnosine (Sigma-Aldrich), and 200 µM α-tocopherol (Sigma-Aldrich) to a serum-free basal medium (Table 16).

**[Table 16]**

| medium used for expansion culture | proliferation fold in 11 days | cell survival rate (%) on day 11 of expansion culture |
|---|---|---|
| IMDM + 15% FBS | 759 | 99.0 |
| X-VIVO-15 | 71 | 97.4 |
| BM220720 + 2 g/L HSA | 210 | 97.3 |
| BM220720 + 2 g/L HSA + 0.3 mM Boric acid + 3 mM Taurine + 5 mg/L LPC + 20 mM carnosine + 200 µM α-Tocopherol | 1600 | 97.7 |

### [Industrial Applicability]

According to the present invention, a cell proliferation maintenance-promotion effect comparable to that by culture in a serum-containing medium can be obtained even when a serum-free medium or low-serum medium is used. In particular, culture in a serum-free medium or low-serum medium that does not require extensive washing is preferred for cells that are vulnerable to a stress caused by operations such as washing of cell and the like, and the cells are suitable for culture under such conditions.

This application is based on a patent application No. 2023-217677 filed in Japan (filing date: December 25, 2023), the contents of which are incorporated in full herein.

## Claims

1. A method for culturing a cell, comprising a step of culturing the cell in a serum-free medium or low-serum medium comprising (i) imidazole dipeptide or a salt thereof, (ii) taurine or a precursor thereof, or a salt thereof, and (iii) lysophosphatidic acid or a derivative thereof.

2. The method according to claim 1, wherein the imidazole dipeptide or a salt thereof is carnosine or anserine, or a salt thereof.

3. The method according to claim 1, wherein the imidazole dipeptide or a salt thereof is carnosine or a salt thereof.

4. The method according to claim 1, wherein the lysophosphatidic acid or a derivative thereof is lysophosphatidylcholine (LPC).

5. The method according to claim 1, wherein the aforementioned medium further comprises vitamin E.

6. The method according to claim 1, wherein the aforementioned medium further comprises boric acid or a salt thereof.

7. The method according to claim 1, wherein the aforementioned serum-free medium or low-serum medium is a serum-free medium.

8. The method according to claim 1, wherein the cell is an immunocompetent cell.

9. The method according to claim 8, wherein the immunocompetent cell is selected from dendritic cell, B cell, T cell, and natural killer cell.

10. The method according to claim 8, wherein the immunocompetent cell is a T cell.

11. The method according to any one of claims 1 to 10, wherein the cell is derived from a pluripotent stem cell.

12. The method according to claim 11, wherein the pluripotent stem cell is an iPS cell.

13. A method for proliferating a cell, comprising a step of culturing the cell in a serum-free medium or low-serum medium comprising (i) imidazole dipeptide or a salt thereof, (ii) taurine or a precursor thereof, or a salt thereof, and (iii) lysophosphatidic acid or a derivative thereof.

14. The method according to claim 13, wherein the imidazole dipeptide or a salt thereof is carnosine or anserine, or a salt thereof.

15. The method according to claim 13, wherein the imidazole dipeptide or a salt thereof is carnosine or a salt thereof.

16. The method according to claim 13, wherein the lysophosphatidic acid or a derivative thereof is lysophosphatidylcholine (LPC).

17. The method according to claim 13, wherein the aforementioned medium further comprises vitamin E.

18. The method according to claim 13, wherein the aforementioned medium further comprises boric acid or a salt thereof.

19. The method according to claim 13, wherein the aforementioned serum-free medium or low-serum medium is a serum-free medium.

20. A method for producing a cell population in which the number of desired cells is expanded, comprising a step of culturing the cell population comprising the desired cells in a serum-free medium or low-serum medium comprising (i) imidazole dipeptide or a salt thereof, (ii) taurine or a precursor thereof, or a salt thereof, and (iii) lysophosphatidic acid or a derivative thereof.

21. The method according to claim 20, wherein the imidazole dipeptide or a salt thereof is carnosine or anserine, or a salt thereof.

22. The method according to claim 20, wherein the imidazole dipeptide or a salt thereof is carnosine or a salt thereof.

23. The method according to claim 20, wherein the lysophosphatidic acid or a derivative thereof is lysophosphatidylcholine (LPC).

24. The method according to claim 20, wherein the aforementioned medium further comprises vitamin E.

25. The method according to claim 20, wherein the aforementioned medium further comprises boric acid or a salt thereof.

26. The method according to claim 20, wherein the aforementioned serum-free medium or low-serum medium is a serum-free medium.

27. A serum-free or low-serum medium composition comprising (i) imidazole dipeptide or a salt thereof, (ii) taurine or a precursor thereof, or a salt thereof, and (iii) lysophosphatidic acid or a derivative thereof.

28. A medicament comprising a cell population obtained by the method according to any one of claims 20 to 26.

29. The medicament according to claim 28, which is for use in the prophylaxis and/or treatment of cancer.

30. A method for the prophylaxis and/or treatment of cancer, comprising administering a cell population obtained by the method according to any one of claims 20 to 26 to a subject in need thereof.

31. A cell population obtained by the method according to any one of claims 20 to 26, which is for use in the prophylaxis and/or treatment of cancer.

32. Use of a cell population obtained by the method according to any one of claims 20 to 26 in the manufacture of a medicament for the prophylaxis and/or treatment of cancer.
